# DEMANDE DE BREVET EUROPEEN

(11) **EP 4 772 541 A1**
(43) Date de publication de la demande: **08.07.2026**
(21) Numéro de dépôt: 25225292.9
(22) Date de dépôt: 18.12.2025
(51) Int. Cl.: C08B 37/00, C08J 3/075, A61K 8/73, A61Q 19/00

(54) **PROCEDE DE RETICULATION DE POLYSACCHARIDES EN MILIEU HYDRO ORGANIQUE CONCENTRE FORMULATIONS TOPIQUES EN COMPRENANT**

(30) Priorité: 07.01.2025 FR 2500129
(71) Demandeur: Société d'Exploitation de Produits pour les Industries Chimiques SEPPIC, 75321 Paris cedex 07 (FR)
(72) Inventeur: BODOC, Miruna, 75321 Paris Cedex 07 (FR); CANDILLE, Hélène, 75321 Paris Cedex 07 (FR); COLAS, Aurélie, 75321 Paris Cedex 07 (FR); GUILBOT, Jérome, 75321 Paris Cedex 07 (FR); MONTEILLET, Stéphane, 75321 Paris Cedex 07 (FR)
(74) Mandataire: Air Liquide

(57) **Abrégé**

L'invention concerne un nouveau procédé de réticulation de polysaccharides d'origine naturelle dans un milieu hydroorganique ainsi que des formulation topiques en comprenant.

## Description

### Domaine technique de l'invention

L'invention relève du domaine des industries cosmétique et pharmaceutique. L'invention concerne plus particulièrement un nouveau procédé de synthèse d'agents épaississants d'origine naturelle.

### État de la technique antérieure

Les polymères sont largement utilisés aujourd'hui dans les formulations à usage topique pour des utilisations dans les domaines de la cosmétique, de la dermocosmétique, de la pharmacie ou de la dermo-pharmacie. La plupart d'entre eux sont des modificateurs de rhéologie, qui épaississent les phases polaires, comme l'eau.

Comme exemples de tels polymères, il y a les polyélectrolytes réticulés, qui ont la capacité de se déployer dans le solvant polaire notamment grâce aux forces de répulsions électrostatiques dues à la présence de charges électriques sur leur squelette polymérique. Ce phénomène permet ainsi de former un réseau polymérique qui induit une augmentation de la viscosité, une consistance et une stabilisation de formulations comme les émulsions de type huile-dans-eau ou eau-dans-huile en présence ou non de tensioactifs émulsionnants ou de gel-crèmes exempts de tensioactifs émulsionnants. Ces polymères, résultent généralement de la polymérisation de monomères de type acrylate ou méthacrylate ou encore de monomères dérivés de l'acrylamide ou de ses dérivés.

Comme polyélectrolytes réticulés commercialisés sur le marché ou divulgués dans la littérature, il y a par exemple :
- L'homopolymère de l'acide acrylique partiellement ou totalement salifié par un sel de sodium, d'ammonium ou de potassium, l'homopolymère de l'acide méthacrylique partiellement ou totalement salifié par un sel de sodium, d'ammonium ou de potassium, l'homopolymère de l'acide 2-méthyl-((1-oxo-2-propényl) amino)-1-propanesulfonique (AMPS) partiellement ou totalement salifié par un sel de sodium, d'ammonium ou de potassium,
- les copolymères de l'acide acrylique, partiellement ou totalement salifié par un sel de sodium, d'ammonium ou de potassium et de l'AMPS, les copolymères de l'acide méthacrylique, partiellement ou totalement salifié par un sel de sodium, d'ammonium ou de potassium et de l'AMPS,
- les copolymères de l'acrylamide et de l'AMPS partiellement ou totalement salifié par un sel de sodium, d'ammonium ou de potassium,
- les copolymères de la vinylpyrolidone et de l'AMPS partiellement ou totalement salifié par un sel de sodium, d'ammonium ou de potassium,
- les copolymères de l'AMPS partiellement ou totalement salifié par un sel de sodium, d'ammonium ou de potassium et de l'acrylate de (2-hydroxy éthyle) ou du méthacrylate de (2-hydroxy éthyle), ou l'acrylate de (2,3-dihydroxy propyle) ou le méthacrylate de (2,3-dihydroxy propyle),
- les copolymères de l'AMPS partiellement ou totalement salifié par un sel de sodium, d'ammonium ou de potassium et de l'hydroxyéthylacrylamide, ou de l'hydroxyéthylmétha-crylamide,
- les copolymères de l'AMPS partiellement ou totalement salifié par un sel de sodium, d'ammonium ou de potassium et du N,N-diméthyl acrylamide, du N,N-diméthyl méthacrylamide, du N,N-diéthyl acrylamide, du N,N-diéthyl méthacrylamide, du N-méthyl acrylamide, du N-éthyl acrylamide, du N-propyl acrylamide, du N-isopropyl acrylamide, du N-butyl acrylamide, du N-(tert-butyl) acrylamide, du N-méthyl méthacrylamide, du N-éthyl méthacrylamide, du N-propyl méthacrylamide, du N-isopropyl méthacrylamide, du N-butyl méthacrylamide, du N-(tert-butyl) méthacrylamide ou du N,N-dipropyl acrylamide,
- les copolymères de l'AMPS partiellement ou totalement salifié par un sel de sodium, d'ammonium ou de potassium et du tris(hydroxyméthyl) acrylamido méthane (THAM),
- les copolymères de l'acide acrylique ou de l'acide méthacrylique, partiellement ou totalement salifiés par un sel de sodium, d'ammonium ou de potassium, et de l'acrylate de (2-hydroxy éthyle), ou l'acrylate de (2,3-dihydroxy propyle),
- les copolymères de l'acide acrylique ou de l'acide méthacrylique, partiellement ou totalement salifiés par un sel de sodium, d'ammonium ou de potassium, et du méthacrylate de (2-hydroxy éthyle), ou le méthacrylate de (2,3-dihydroxy propyle),
- les copolymères de l'acide acrylique ou de l'acide méthacrylique partiellement ou totalement salifiés par un sel de sodium, d'ammonium ou de potassium, et de l'hydroxyéthylacry-lamide,
- les copolymères de l'acide acrylique ou de l'acide méthacrylique partiellement ou totalement salifiés par un sel de sodium, d'ammonium ou de potassium et du THAM,
- les copolymères de l'acide acrylique ou de l'acide méthacrylique partiellement ou totalement salifiés par un sel de sodium, d'ammonium ou de potassium et du N,N-diméthyl acrylamide, du N,N-diméthyl méthacrylamide, du N,N-diéthyl acrylamide, du N,N-diéthyl méthacrylamide, du N-méthyl acrylamide, du N-éthyl acrylamide, du N-propyl acrylamide, du N-isopropyl acrylamide, du N-butyl acrylamide, du N-(tert-butyl) acrylamide, du N-méthyl méthacrylamide, du N-éthyl méthacrylamide, du N-propyl méthacrylamide, du N-isopropyl méthacrylamide, du N-butyl méthacrylamide, du N-(tert-butyl)méthacrylamide, ou du N,N-di-propyl acrylamide,
- les terpolymères branchés ou réticulés de l'acide acrylique ou de l'acide méthacrylique partiellement ou totalement salifiés par un sel de sodium, d'ammonium ou de potassium, de l'AMPS partiellement ou totalement salifié par un sel de sodium, d'ammonium ou de potassium et de l'acrylate de (2-hydroxy éthyle), du méthacrylate de (2-hydroxy éthyle), de l'acrylate de (2,3-dihydroxy propyle) ou du méthacrylate de (2,3-dihydroxy propyle),
- les terpolymères branchés ou réticulés de l'acide acrylique ou de l'acide méthacrylique partiellement ou totalement salifiés par un sel de sodium, d'ammonium ou de potassium, de l'AMPS partiellement ou totalement salifié par un sel de sodium, d'ammonium ou de potassium et du THAM,
- les terpolymères branchés ou réticulés de l'acide acrylique ou de l'acide méthacrylique partiellement ou totalement salifiés par un sel de sodium, d'ammonium ou de potassium, de l'AMPS partiellement ou totalement salifié par un sel de sodium, d'ammonium ou de potassium et du N,N-diméthyl acrylamide, du N,N-diméthyl méthacrylamide, du N,N-diéthyl acrylamide, du N,N-diéthyl méthacrylamide, du N-méthyl acrylamide, du N-éthyl acrylamide, du N-propyl acrylamide, du N-isopropyl acrylamide, du N-butyl acrylamide, du N-(tert-butyl) acrylamide, du N-méthyl méthacrylamide, du N-éthyl méthacrylamide, du N-propyl méthacrylamide, du N-isopropyl méthacrylamide, du N-butyl méthacrylamide, du N-(tert-butyl)méthacrylamide, ou du N,N-dipropyl acrylamide,
- les terpolymères branchés ou réticulés de l'acide acrylique ou de l'acide méthacrylique partiellement ou totalement salifiés par un sel de sodium, d'ammonium ou de potassium, de l'AMPS partiellement ou totalement salifié par un sel de sodium, d'ammonium ou de potassium et de l'acrylamide ou du méthacrylamide,
- les copolymères de l'acide acrylique ou de l'acide méthacrylique partiellement ou totalement salifiés par un sel de sodium, d'ammonium ou de potassium, et d'acrylates d'alkyle ou de méthacrylate d'alkyle dont la chaîne carbonée comprend entre quatre et trente atomes de carbone et plus particulièrement entre dix et trente atomes de carbone, et
- les copolymères de l'AMPS partiellement ou totalement salifiés par un sel de sodium, d'ammonium ou de potassium et d'acrylates d'alkyle ou de méthacrylate d'alkyle dont la chaîne carbonée comprend entre quatre et trente atomes de carbone et plus particulièrement entre dix et trente atomes de carbone.

Aujourd'hui ces polymères, qu'ils soient sous forme de latex inverses auto-inversibles, de latex inverses concentrés ou de poudres, permettent de répondre aux besoins clients en termes de performances épaississantes, dans un solvant polaire comme l'eau. Les gels aqueux obtenus par leur dispersion dans ce solvant, présentent un aspect lisse, exempts de grains ou grumeaux, avec des propriétés sensorielles particulières au toucher, ainsi qu'une facilité de préhension et d'application sur la peau.

Malgré l'offre commerciale importante, la demande de nouveaux épaississants de phases aqueuses pour la formulation de produits cosmétiques et dermopharmaceutiques reste d'actualité car les polymères utilisés aujourd'hui sont majoritairement d'origine pétrochimique, alors que les industries utilisatrices sont engagées dans une démarche d'éco-conception. Il est donc nécessaire de développer des épaississants alternatifs qui, tout en ayant des propriétés comparables, ont un profil environnemental amélioré, soit par leur origine ou celle de leurs précurseurs, soit par leur caractère biodégradable. L'utilisation de polysaccharides semble constituer une solution alternative acceptable car ils sont déjà utilisés depuis de nombreuses années comme agents modificateurs de la texture ou de la rhéologie dans l'industrie pour préparer des produits pharmaceutiques, cosmétiques ou alimentaires.

Selon leur constitution chimique, ils peuvent être utilisés comme agents gélifiants ou comme agents épaississants. Par agent épaississant, on entend un composé chimique qui augmente la viscosité du milieu dans lequel il est introduit. Par agent gélifiant, on entend un composé qui transforme un milieu liquide en un état structuré, qui ne coule pas, par formation d'un réseau tridimensionnel au sein du liquide ; le gel étant considéré comme un état intermédiaire entre l'état liquide et l'état solide.

Les polysaccharides sont des polymères de saccharides ou plus couramment de sucres. Selon la nomenclature de l'Union internationale de chimie pure et appliquée (IUPAC), par saccharides, on désigne des oses, des composés d'oses proprement dits et leurs dérivés obtenus soit par réduction d'un groupement carbonyle, soit par oxydation d'une ou de plusieurs fonctions hydroxyle, soit par le remplacement d'une ou de plusieurs fonctions hydroxyles par un atome d'hydrogène, ou une fonction amine, phosphate sulfate.

Les polysaccharides les plus couramment utilisés pour les industries susmentionnées sont majoritairement choisis parmi les polymères contenant des unités monomériques d'oses tels que le glucose, le galactose, le mannose, le xylose, l'arabinose ou des unités monomériques de dérivés d'oses dans lesquels la fonction hydroxyle du carbone terminal a été oxydée en fonction carboxyle.

Parmi les polysaccharides constitués uniquement d'oses (polyoses), il y a notamment :
- l'amidon et les dérivés d'amidon qui est un homopolymère du glucose comportant des liaisons glycosidiques alpha-1,4 ; on distingue l'homopolymère linéaire (liaisons alpha-1,4 uniquement) appelé amylose (environ 20% de l'amidon) et un homopolymère ramifié (liaisons glycosidiques alpha-1,4 et alpha-1,6) appelé amylopectine (environ 80% de l'amidon). L'amidon est obtenu à partir de végétaux comme le blé, le maïs ou la pomme de terre ;
- La cellulose qui est un homopolymère de glucose comportant liaisons glycosidiques béta-1,4 ; La cellulose est extraite du bois et est principalement utilisée dans l'industrie papetière pour fabriquer de la pâte à papier.
- L'hémicellulose qui est un polymère de différents sucres comme le glucose, le mannose, le galactose, le xylose, l'arabinose, le rhamnose, le xylose étant souvent majoritaire et l'hémicellulose comportant aussi parfois des acides uroniques.

Parmi les polysaccharides constitués de dérivés d'oses, il y a notamment :
- Les galactanes sulfatés qui sont des polymères du galactose pouvant avoir des groupements esters-sulfate appendus, tels que les polyosides algaux ou l'agar ;
- Les uronanes qui sont les polymères d'acides uroniques comme les algines et les pectines,
- Les hétéropolymères d'oses parmi lesquelles il y a les galactomannanes, comme les gommes de guar, de tara, de caroube et de fenugrec et les glucomannoglycanes comme la gomme de Konjac et
- les xyloglycanes comme la gomme de Tamarin,
- les hétéropolymères d'oses et d'acides uroniques ; ces polymères se trouvent notamment dans les exsudats de sève, comme les exsudats des gommes arabique et de karaya ; ils sont aussi produits aussi par des micro-organismes comme les gommes xanthane et gellane,
- les glucosaminoglycanes : ce sont des polyosides formés à partir du glucose par remplacement de son hydroxyle sur C-2 par une fonction amine (appelé 2-amino-2-désoxy-D-glucose ou glucosamine) ; La fonction amine peut être acétylée. Parmi les polysaccharides de cette classe, on trouve le chitosan formé uniquement de motifs glucosamine, la chitine avec les fonctions amines acétylées et l'hyaluronane dont l'unité de répétition est un dimère de glucosamine et d'acide glucuronique.

La modification chimique de polysaccharides constitue une voie permettant d'accroître leurs performances initiales et d'apporter également de nouvelles propriétés. Il s'agit soit de la fonctionnalisation chimique par greffage de nouveaux groupements chimiques de poids moléculaires plus ou moins importants, soit de la réticulation qui consiste à associer les chaînes polysaccharidiques entre elles au moyen d'un agent de réticulation au moins difonctionnel appelé réticulant. Dans les deux cas, les fonctions des polysaccharides de départ impliqués restent les groupes hydroxyle (oses), amino (dérivés de glucosamine) ou carboxyliques (dérivés d'acides uroniques).

Cependant, de telles fonctionnalisations sont souvent réalisées avec des réactifs peu respectueux de l'environnement ou selon des modes opératoires ne respectant que partiellement les douze principes de la chimie verte tels que l'utilisation de solvants organiques. De plus, les produits correspondants obtenus ne permettent pas aujourd'hui de concurrencer en termes de performance épaississante ou gélifiante, les polymères épaississants d'origine pétrochimique commercialisés.

Parmi les agents réticulants couramment utilisés pour la réticulation de polysaccharides naturels, seuls ceux appartenant à la famille de dérivés polyphosphatés comme le triméthaphosphate de sodium (STMP) ou le tripolyphosphate de sodium (STPP), sont intéressants d'un point de vue environnemental. Le (STMP) est un composé non toxique pour l'homme, couramment utilisé dans les industries alimentaire et pharmaceutique ; Il est synthétisé par déshydratation à haute température du polyphosphate de sodium ; il est partiellement soluble dans l'eau froide, très peu soluble dans l'eau chaude, insoluble dans le méthanol, le diéthyl éther, le n-octanol ou l'acétone. La réticulation de polysaccharides avec le (STMP), décrite dans la littérature brevet ou académique, est effectuée en milieu aqueux, dans des conditions de pH basique, à une température entre 20°C et 50°C pendant plusieurs heures.

Cependant, les procédés de réticulation mis en œuvre jusqu'à aujourd'hui, conduisent à des gels de polymère naturel réticulés très dilués, soit environ à 3% massique de polymère réticulé. Une telle dilution ne permet pas de préparer à l'échelle industrielle des polysaccharides naturels réticulés sous forme de poudres. D'autre part, la commercialisation d'un tel hydrogel, à savoir une solution comprenant de l'eau et du polysaccharide réticulé à une teneur massique de 3% ou de 5%, rendrait nécessaire l'utilisation d'un agent conservateur qui serait *de* facto imposé au formulateur de compositions cosmétiques ou pharmaceutiques à usage topique, et pourrait se révéler incompatible avec le propre système conservateur desdites compositions à usage topique. Il existe donc un besoin de développer un procédé de réticulation qui conduise à un gel de polymère d'origine naturelle soit plus concentré soit dénué d'eau ajoutée, et se présentant sous la forme d'une poudre.

### EXPOSE DE L'INVENTION

C'est la raison pour laquelle, selon un premier aspect l'invention a pour objet un procédé de préparation d'au moins un polysaccharide réticulé comprenant les étapes suivantes ;
- Une étape a) de préparation d'un mélange eau - solvant polaire, ledit solvant polaire étant choisi dans le groupe constitué par les alcools aliphatiques comportant de un à quatre atomes de carbone, des cétones comportant de trois à cinq atomes de carbone et les polyols comportant de deux ou trois groupes hydroxyle et de deux à six atomes de carbone ladite préparation étant effectuée en mélangeant l'eau et ledit solvant polaire dans des proportions massiques telles que le rapport massique solvant polaire sur eau est supérieur ou égal à 0,4 et inférieur ou égal à 19,0 ;
   - Une étape b) de dispersion d'au moins un polysaccharide dans ledit mélange eau - solvant polaire préparé à l'étape a), pour obtenir un milieu réactionnel comprenant pour 100% de sa masse, une proportion massique en polysaccharide supérieure à 10% massique et inférieure ou égale à 55% massique, ledit au moins un polysaccharide mis en œuvre à l'étape b), est choisi dans le groupe consti-tué par la gomme xanthane, le xanthane greffé avec des chaines hydrocarbonées comportant de deux à vingt-deux atomes de carbone et plus particulièrement, la gomme xanthane estérifiée par de l'acide dodécanoïque, les gommes de guar et de konjac, les carraghénanes et plus particulièrement, le kappa-carraghénane et l'iota-carraghénane et des mélanges de deux ou de plusieurs desdits polysaccharides de ce groupe ;
- Une étape c) d'ajustement du pH du milieu réactionnel préparé à l'étape b) à une valeur supérieure ou égale à 8,0 et inférieure ou égale à 13,0 et plus particulièrement supérieure ou égale à 8,5 et inférieure ou égale à 12,5 en y ajoutant une base ;
- Une étape d) de réticulation dudit au moins un polysaccharide par addition dans le milieu réactionnel basique obtenu à l'issue de l'étape c), d'un agent de réticulation phosphate de choisi dans le groupe constitué du trimétaphosphate de sodium (STMP) et du tripolyphosphate de sodium (STPP), pour obtenir une dispersion basique comprenant du polysaccharide réticulé ;
   - - Une étape e) d'ajustement du pH de ladite dispersion basique obtenue l'issue de l'étape d), à une valeur inférieure ou égale à 7,0 pour obtenir une dispersion non-basique dudit polysaccharide réticulé ;
- Une étape f) de filtration de ladite dispersion non-basique obtenue à l'issue de l'étape e) pour en récupérer ledit au moins un polysaccharide réticulé attendu ; optionnellement suivie :
- Soit d'une étape g) de séchage pour en éliminer les traces de solvants résiduels,
- Soit d'une étape h) d'atomisation dudit au moins un polysaccharide réticulé obtenu à l'étape f) pour en obtenir une poudre.

Selon un aspect particulier du procédé tel que défini ci-dessus, les étapes a) et b) sont simultanées et constituent une seule étape A) de préparation d'un milieu réactionnel par mélange d'eau, d'un solvant polaire choisi dans le groupe constitué par les alcools aliphatiques comportant de un à quatre atomes de carbone, des cétones comportant de trois à cinq atomes de carbone et les polyols comportant de deux ou trois groupes hydroxyle et de deux à six atomes de carbone et d'un polysaccharide en des proportions telles que
- la proportion massique en ledit au moins un polysaccharide est supérieure à 10% massique et inférieure ou égale à 55% massique dudit milieu réactionnel et que
- le rapport massique solvant polaire sur eau dudit mélange est supérieur ou égal à 0,4 et inférieur ou égal à 19,0.

Dans le procédé tel que défini ci-dessus, le solvant polaire du mélange préparé à l'étape a) ou dudit milieu réactionnel préparé à l'étape A), est plus particulièrement choisi dans le groupe constitué par le méthanol, l'éthanol, le butanol, l'isopropanol, l'acétone, la méthyl éthyl cétone (MEK), le glycérol, le 1,3-propanediol, le butylèneglycol, le 1,3-butanediol, le pentylène glycol l'hexylène glycol et le 2-méthyl 2,4-pentanediol ; selon ce mode particulier, ledit solvant polaire est tout particulièrement choisi dans le groupe constitué par l'éthanol et l'isopropanol.

Dans le procédé tel que défini ci-dessus, un ou plusieurs sels sont optionnellement ajoutés lors de la préparation du mélange eau - solvant préparé à l'étape a) ou dudit milieu réactionnel préparé à l'étape A), par exemple un sel choisi dans le groupe constitué du chlorure de sodium, du chlorure de calcium, du chlorure de magnésium, du sulfate de calcium, du sulfate de magnésium, du carbonate de calcium, du carbonate de magnésium, de l'aspartate de magnésium et autres sels monovalents ou divalents acceptables dans les industries cosmétique pharmaceutique phytosanitaire ou alimentaire.

Dans le procédé tel que défini ci-dessus, le rapport massique solvant polaire sur eau dudit mélange préparé à l'étape a) ou dudit milieu réactionnel préparé à l'étape A), est plus particulièrement supérieur ou égal à 1,0 et inférieur ou égal à 4,0.

Dans le procédé tel que défini ci-dessus, le milieu réactionnel préparé à l'étape b) ou à l'étape A) comprend plus particulièrement pour 100% de sa masse, une proportion massique en ledit au moins un polysaccharide supérieure ou égale à 15% et inférieure ou égale à 45% massique.

Selon un aspect particulier de la présente invention, si nécessaire, l'étape b) de solubilisation ou de dispersion dudit au moins un polysaccharide ou l'étape A) de préparation du dit milieu réactionnel est effectuée à une température comprise entre 50°C et 100°C, préférentiellement entre 60°C et 80°C.

Dans le procédé tel que défini ci-dessus, l'étape c), Elle est effectuée en ajoutant au mélange réactionnel préparé à l'étape b) ou l'étape A), une base alcaline, comme l'hydroxyde de sodium ou l'hydroxyde de potassium, l'ammoniaque, ou une base aminée. On utilise plus particulièrement la soude et notamment une solution aqueuse de soude tétramolaire, la potasse, l'ammoniaque ou la triéthylamine. Dans le procédé tel que défini ci-dessus, à l'étape c), le pH est plus particulièrement ajusté à une valeur supérieure ou égale à 10,0 et inférieure ou égal à 12,5.

Dans le procédé tel que défini ci-dessus, le rapport massique réticulant STMP ou STPP mis en œuvre à l'étape d) sur ledit au moins un polysaccharide de départ est supérieur ou égal à 0,0001 et inférieur ou égal à 0,0700 ; il est plus particulièrement supérieur ou égal à 0,0003 et inférieur ou égal à 0,0300. La température de réticulation peut varier entre 5°C et 100°C, préférentiellement entre 10°C et 80°C et idéalement entre 20°C et 70°C.

Selon un autre mode particulier du procédé tel que défini ci-dessus, l'agent de réticulation mis en œuvre à l'étape d) est le trimétaphosphate de sodium (STMP).

Dans le procédé tel que défini ci-dessus, l'étape e) d'ajustement du pH est notamment effectuée un acide fort comme l'acide chlorhydrique ou l'acide sulfurique, pour arrêter le mécanisme de réticulation. Elle est généralement effectuée à température ambiante, entre 15°C et 35°C.

Le procédé tel que défini ci-dessus permet d'obtenir des gels de polysaccharides plus concentrés, voire sous forme solide, ce qui rend industriellement possible leur utilisation en dermocosmétique et en dermopharmacie comme substitut des polymères d'origine pétrolière.

C'est pourquoi l'invention a aussi pour objet l'utilisation d'un polysaccharide réticulé ou d'un mélange de polysaccharides réticulés tel qu'obtenus par le procédé tel que défini ci-dessus en vue d'épaissir de stabiliser ou d'émulsionner une formulation topique cosmétique ou pharmaceutique. Elle a encore pour objet l'utilisation du polysaccharide réticulé tel qu'obtenu par le procédé tel que défini ci-dessus en vue de suspendre des particules solides dans une formulation topique cosmétique ou pharmaceutique.

C'est pourquoi l'invention a aussi pour objet une formulation topique cosmétique ou pharmaceutique caractérisée en ce qu'elle comprend pour 100% de sa masse, de 0,1% à10,0% et plus particulièrement de 0,5% à 5,0% d'un polysaccharide réticulé ou d'un mélange de polysaccharides réticulés tel qu'obtenus par le procédé tel que défini précédemment, comme agent épaississant, comme agent stabilisant ou comme agent émulsionnant de la dite formulation topique cosmétique ou pharmaceutique ou comme agent apte et destiné à suspendre des particules solides au sein de ladite formulation topique cosmétique ou pharmaceutique.

Les particules solides suspendues dans la formulations topique cosmétique ou pharmaceutique objet de l'utilisation ou de l'invention telle que définie ci-dessus, ont une géométrie régulière ou irrégulière, et se présentent généralement sous forme de perles, de billes, de tiges, de paillettes, de lamelles ou de polyèdres. Elles se caractérisent par un diamètre moyen apparent compris entre un micromètre et cinq millimètres, plus particulièrement entre dix micromètres et un millimètre. Comme exemples de telles particules solides, il y a il y a les micas, l'oxyde de fer, l'oxyde de titane, l'oxyde de zinc, l'oxyde d'aluminium, le talc, la silice, le kaolin, les argiles, le nitrure de bore, le carbonate de calcium, le carbonate de magnésium, l'hydrogénocarbonate de magnésium, les pigments colorés inorganiques, les polyamides comme le nylon-6, les polyéthylènes, les polypropylènes, les polystyrènes, les polyesters, les polymères acryliques ou méthacryliques comme les polyméthylméthacrylates, le polytétrafluoroéthylène, les cires cristallines ou microcristallines, des sphères poreuses, le sulfure de sélénium, le pyrithione de zinc, les amidons, les alginates, les fibres de végétaux, les particules de Loofah ou les particules d'éponges.

Dans la formulation topique cosmétique ou pharmaceutique objet de l'utilisation ou de l'invention telle que définie ci-dessus, l'adjectif topique signifie que ladite formulation est mise en œuvre par application sur la peau, les cheveux, le cuir chevelu ou les muqueuses, qu'il s'agisse d'une application directe ou d'une application indirecte lorsque la composition topique selon l'invention est imprégnée sur un support destine à être mis en contact avec la peau (papier, lingette, textile, dispositif transdermique, etc.).

La formulation topique cosmétique ou pharmaceutique objet de l'utilisation ou de l'invention telle que définie ci-dessus, peut se présenter sous toute forme physique, par exemple sous la forme d'un gel aqueux hydro-alcoolique ou hydro-glycolique; d'une solution; d'une poudre; d'une suspension, d'une émulsion, d'une microémulsion ou d'une nano-émulsion, qu'elles soient de type eau-dans-huile, huile-dans-eau, eau-dans-huile-dans-eau ou huile-dans-eau-dans-huile. Elle peut être conditionnée dans un flacon, dans un dispositif de type "flacon" pompe, sous forme pressurisée dans un dispositif aérosol, dans un dispositif muni d'une paroi ajourée comme une grille ou dans un dispositif muni d'un applicateur à bille (dit "roll-on").

De façon générale, la formulation topique cosmétique ou pharmaceutique objet de l'utilisation ou de l'invention telle que définie ci-dessus, peut également comprendre un ou plusieurs composants ingrédients cosmétiquement ou pharmaceutiquement acceptables, qu'il s'agisse de principes actifs ou d'excipients comme les tensioactifs moussants, non moussants ou détergents, les agents épaississants ou gélifiants, les agents stabilisants, les agents solubilisants, les composés filmogènes, les solvants et co-solvants, les agents hydrotropes, les eaux thermales ou minérales les agents plastifiants, les agents émulsionnants et co-émulsionnants, les agents opacifiants, les agents nacrant, les agents surgraissants, les séquestrants, les agents chélatants, les huiles, les cires, les agents antioxydants, les parfums, les huiles essentielles, les agents conservateurs, les agents conditionneurs, les agents déodorants, les agents blanchissants destines à la décoloration des poils et de la peau, les principes actifs destinés à apporter une action traitante ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

L'expression « cosmétiquement acceptable » utilisée dans la définition des ingrédients susmentionnés, signifie selon la directive du Conseil de la Communauté Economique Européenne N° 76/768/CEE du 27 juillet 1976 modifiée par la directive N°93/35/CEE du 14 juin 1993, que ledit excipient (E) comprend de l'eau et toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect ou d'en corriger les odeurs corporelles ou de les protéger ou de les maintenir en bon état.

L'expression « pharmaceutiquement acceptable » utilisée dans la définition des ingrédients susmentionnés, signifie que ces ingrédients sont inscrits dans la Pharmacopée de l'Etat dans lequel ladite formulation est utilisée.

Comme exemples de tensioactifs moussants, optionnellement présents dans la formulation topique cosmétique ou pharmaceutique objet de l'utilisation ou de l'invention telles que définies ci-dessus, il y a les tensioactifs moussants ou détergents anioniques, cationiques, amphotères ou non ioniques.

Parmi les tensioactifs anioniques moussants, on peut citer les sels de métaux alcalins, les sels de métaux alcalino-terreux, les sels d'ammonium, les sels d'amines ou les sels d'amino-alcools :
- des alkyl éthers sulfates, comme le C12-14 myristyl éther sulfate de sodium à 3,5 moles d'oxyde d'éthylène, le C12-14 lauryl éther sulfate de sodium à 2 moles d'oxyde d'éthylène, le C12-14 lauryl éther sulfate d'ammonium à 2 moles d'oxyde d'éthylène, le C12-14 lauryl éther sulfate de mono isopropyl amine à 2 moles d'oxyde d'éthylène dans le propylène glycol, le C12-14 lauryl éther sulfate de tri isopropyl amine à 2 moles d'oxyde d'éthylène dans le propylène glycol, le C12-14 lauryl éther sulfate de sodium à 3 moles d'oxyde d'éthylène, le C12-14 lauryl éther sulfate d'ammonium à 3 moles d'oxyde d'éthylène, le C12-15 lauryl éther sulfate de sodium à 3 moles d'oxyde d'éthylène, le C8-10 lauryl éther sulfate de sodium à 3 moles d'oxyde d'éthylène, le C8-10 lauryl éther sulfate d'ammonium à 3 moles d'oxyde d'éthylène, le C9-11 lauryl éther sulfate de sodium à 2,5 moles d'oxyde d'éthylène, le C9-11 lauryl éther sulfate d'ammonium à 2,5 moles d'oxyde d'éthylène, le C9-11 lauryl éther sulfate d'ammonium à 2,5 moles d'oxyde d'éthylène dans l'hexylène glycol ;
- des alkylsulfates comme le lauryl sulfate de sodium, le lauryl sulfate de potassium, le lauryl sulfate d'ammonium, le lauryl sulfate de magnésium, le cocoyl sulfate de sodium, le cocoyl sulfate de potassium, le cocoyl sulfate d'ammonium, le cocoyl sulfate de magnésium ;
- des alkylamidoéthersulfates,des alkylarylpolyéthersulfates, des monoglycérides sulfates, des alpha-oléfinesulfonates, des paraffines sulfonates ;
- des alkylphosphates, des alkylétherphosphates, des alkylsulfonates, des alkylamides sulfonates, des alkylarylsulfonates, des alkylcarboxylates, des alkylsulfosuccinates, des alkyléthersulfosuccinates, des alkylamidesulfosuccinates, des alkylsulfoacétates ;
- des dérivés N-acylés d'acides aminés, comme le lauroyl sarcosinate de sodium, le lauroyl sarcosinate de potassium, le lauroyl sarcosinate de magnésium, le lauroyl sarcosinate d'ammonium, le lauroyl glycinate de sodium, le lauroyl glycinate de potassium, le lauroyl glycinate de magnésium, le lauroyl glycinate d'ammonium, le cocoyl sarcosinate de sodium, le cocoyl sarcosinate de potassium, le cocoyl sarcosinate de magnésium, le cocoyl sarcosinate d'ammonium, le cocoyl glycinate de sodium, le cocoyl glycinate de potassium, le cocoyl glycinate de magnésium, le cocoyl glycinate d'ammonium, le cocoyl glutamate de sodium, le cocoyl glutamate de potassium, le cocoyl glutamate de magnésium, le cocoyl glycinate d'ammonium, le cocoyl aspartate de sodium, le cocoyl aspartate de potassium, le cocoyl aspartate de magnésium, le cocoyl aspartate d'ammonium ; ou des mélanges comprenant lesdits dérivés N-acylés d'acides aminés comme ceux par exemple commercialisés sous le nom de marque Proteol^{™}OAT, Proteol^{™}APL, Oramix^{™}L30 ;
- des acyliséthionates, comme le cocoyl iséthionate de sodium, le cocoyl iséthionate de potassium, le cocoyl iséthionate de magnésium, le cocoyl iséthionate d'ammonium, le lauroyl iséthionate de sodium, le lauroyl iséthionate de potassium, le lauroyl iséthionate de magnésium, le lauroyl iséthionate d'ammonium ;
- des N-acyltaurates, comme le méthyl cocoyl taurate de sodium, le méthyl cocoyl taurate de potassium, le méthyl cocoyl taurate de magnésium, le méthyl cocoyl taurate d'ammonium ou
- des acyllactylates.

Parmi les tensioactifs amphotères moussants, on peut citer :
- les alkylbétaïnes, comme la lauryl bétaïne, la cocoyl bétaïne, la myristyl bétaïne ;
- les alkylamidobétaïnes, comme la lauramidopropyl bétaïne, le sel de sodium de la cocamidopropyl bétaïne, la 1-propanaminium, 3-amino-N-(carboxyméthyl)-N,N-diméthyl-, ou la composition commercialisée sous le nom de marque Amonyl^{™}380 BA ;
- les sultaïnes, les alkylamidoalkylsulfobétaïnes, ou la composition commercialisée sous le nom de marque Amonyl^{™}675 SB ou
- les dérivés d'imidazolines, les phosphobétaïnes, les amphopolyacétates et les ampho-propionates.

Parmi les tensioactifs cationiques moussants, on peut citer particulièrement les dérivés d'ammoniums quaternaires.

Parmi les tensioactifs non ioniques moussants, on peut citer plus particulièrement les alkylpolyglycosides comportant un radical aliphatique, linéaire ou ramifié, saturé ou insaturé, et comportant de 8 à 16 atomes de carbone, comme le n-octyl polyglucoside, le n-décyl polyglucoside, n-undécylényl polyglucoside, le n-dodécyl polyglucoside, le n-tétradécyl polyglucoside, le n-hexadécyl polyglucoside, le 1-12 dodécanediyl polyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines ;

Comme exemples d'agents épaississants ou gélifiants optionnellement présents dans la formulation topique cosmétique ou pharmaceutique objet de l'utilisation ou de l'invention telles que définies ci-dessus, on peut citer :
- les esters gras d'alkylpolyglycosides éventuellement alcoxylés, comme les esters de méthylpolyglucoside éthoxylés tels que le PEG 120 méthyl glucose trioléate et le PEG 120 méthyl glucose dioléate commercialisés respectivement sous les appellations GLUCAMATE^{™} LT et GLUMATE^{™} DOE120 ;
- les esters gras alcoxylés tels que le PEG 150 pentaérythrytyl tétrastéarate commercialisé sous l'appellation CROTHIX^{™} DS53,
- le PEG 55 propylène glycol oléate commercialisé sous l'appellation ANTIL^{™} 141,
- les carbamates de polyalkylène glycols à chaînes grasses comme le PPG-14 laureth iso-phoryl dicarbamate commercialisé sous l'appellation ELFACOS^{™} T211, ou le PPG-14 palmeth-60 hexyl dicarbamate commercialisé sous l'appellation ELFACOS^{™} GT2125 ;
- la cellulose, les dérivés de la cellulose comme la méthyl-cellulose, l'éthyl-cellulose, l'hydroxy-éthyl cellulose, l'hydroxy-propyl cellulose, l'hydroxy-propyl méthyl cellulose, la carboxy méthyl cellulose,
- l'amidon, les dérivés hydrophiles de l'amidon,
- les polyuréthanes,
- les silicates et les phyllosilicates comme les silicates d'aluminium, les silicates de magnésium, aluminium ou de magnésium, la kaolinite, la montmorillonite, l'illite, la beidellite, la saponite, la bentonite, l'hectorite, la vermiculite, la serpentine, la nacrite, l'amésite, la nontronite, la lizardite, la beidellite, la séricite, l'halloylsite, la muscovite, la paragonite, la damouzite, la glauconite ou la céladonite ;
- les polyélectrolytes branchés ou réticulés, anioniques, cationiques ou amphotères comme les homopolymères branchés ou réticulés de l'acide acrylique, de l'acide méthacrylique ou de l'acide 2-méthyl-[(1-oxo-2-propényl)amino]-1-propanesulfonique (AMPS) partiellement ou totalement salifiés par un sel de sodium, d'ammonium ou de potassium ; les copolymères de l'acide acrylique ou de l'acide méthacrylique, partiellement ou totalement salifiés par un sel de sodium, d'ammonium ou de potassium et de l'AMPS partiellement ou totalement salifié par un sel de sodium, d'ammonium ou de potassium ; les copolymères branchés ou réticulés de l'acrylamide ou de la vinylpyrolidone et de l'AMPS partiellement ou totalement salifié par un sel de sodium, d'ammonium ou de potassium ; les copolymères branchés ou réticulés de l'AMPS partiellement ou totalement salifié par un sel de sodium, d'ammonium ou de potassium et de l'acrylate de (2-hydroxyéthyle), du méthacrylate de (2-hydroxyéthyle), de l'acrylate de (2,3-dihydroxy propyle) ou du méthacrylate de (2,3-dihydroxy propyle), de l'hydroxyéthylacrylamide, de l'hydroxyéthylméthacrylamide, du N,N-diméthyl acrylamide, du N,N-diméthyl méthacrylamide, du N,N-diéthyl acrylamide, du N,N-diéthyl méthacrylamide, du N-méthyl acrylamide, du N-éthyl acrylamide, du N-propyl acrylamide, du N-isopropyl acrylamide, du N-butyl acrylamide, du N-(tert-butyl) acrylamide, du N-méthyl méthacrylamide, du N-éthyl méthacrylamide, du N-propyl méthacrylamide, du N-isopropyl méthacrylamide, du N-butyl méthacrylamide, du N-(tert-butyl)méthacrylamide, du N,N-dipropyl acrylamide ou du tris(hydroxyméthyl)acrylamido méthane (THAM) ; les copolymères branchés ou réticulés de l'acide acrylique ou de l'acide méthacrylique, partiellement ou totalement salifiés par un sel de sodium, d'ammonium ou de potassium et de l'acrylate de (2-hydroxyéthyle), du méthacrylate de (2-hydroxyéthyle), de l'acrylate de (2,3-dihydroxy propyle), du méthacrylate de (2,3-dihydroxy propyle), de l'hydroxyéthylacrylamide, de l'hydroxyéthylméthacrylamide, du N,N-diméthyl acrylamide, du N,N-diméthyl méthacrylamide, du N,N-diéthyl acrylamide, du N,N-diéthyl méthacrylamide, du N-méthyl acrylamide, du N-éthyl acrylamide, du N-propyl acrylamide, du N-isopropyl acrylamide, du N-butyl acrylamide, du N-(tert-butyl) acrylamide, du N-méthyl méthacrylamide, du N-éthyl méthacrylamide, du N-propyl méthacrylamide, du N-isopropyl méthacrylamide, du N-butyl méthacrylamide, du N-(tert-butyl)méthacrylamide, du N,N-dipropyl acrylamide ou du tris(hydroxyméthyl)acrylamido méthane (THAM) ; les terpolymères branchés ou réticulés de l'acide acrylique ou de l'acide méthacrylique partiellement ou totalement salifiés par un sel de sodium, d'ammonium ou de potassium, de l'AMPS partiellement ou totalement salifié par un sel de sodium, d'ammonium ou de potassium et de l'acrylate de (2-hydroxy éthyle), du méthacrylate de (2-hydroxy éthyle), de l'acrylate de (2,3-dihydroxy propyle) du méthacrylate de (2,3-dihydroxy propyle), de l'hydroxyéthylacrylamide, de l'hydroxyéthylméthacrylamide, du N,N-diméthyl acrylamide, du N,N-diméthyl méthacrylamide, du N,N-diéthyl acrylamide, du N,N-diéthyl méthacrylamide, du N-méthyl acrylamide, du N-éthyl acrylamide, du N-propyl acrylamide, du N-isopropyl acrylamide, du N-butyl acrylamide, du N-(tert-butyl) acrylamide, du N-méthyl méthacrylamide, du N-éthyl méthacrylamide, du N-propyl méthacrylamide, du N-isopropyl méthacrylamide, du N-butyl méthacrylamide, du N-(tert-butyl)méthacrylamide, du N,N-dipropyl acrylamide du tris(hydroxyméthyl)acrylamido méthane (THAM) ; de l'acrylamide, ou du méthacrylamide ; les copolymères branchés ou réticulés de l'acide acrylique, de l'acide méthacrylique ou de l'AMPS partiellement ou totalement salifiés par un sel de sodium, d'ammonium ou de potassium et d'un acrylate d'alkyle ou d'un méthacrylate d'alkyle dont la chaîne carbonée comprend entre quatre et trente atomes de carbone et plus particulièrement entre dix et trente atomes de carbone ; les terpolymères branchés ou réticulés de l'AMPS partiellement ou totalement salifiés par un sel de sodium, d'ammonium ou de potassium, avec au moins un monomère neutre, et au moins un monomère de formule CH₂=C(R'₃)-C(=O)-[CH₂-CH₂-O]ₙ-R' dans laquelle R'₃ représente un atome d'hydrogène ou un radical méthyle, R'₄ représente un radical alkyle linéaire ou ramifié saturé ou insaturé comportant de huit à trente atomes de carbone et plus particulièrement un radical choisi parmi les éléments du groupe constitué par le radical octyle, décyle, undécyle, dodécyle, tridécyle, tétradécyle, pentadécyle, hexadécyle, heptadécyle, octadécyle, nonadécyle, eicosyle, 2-éthyl hexyle, 2-propyl heptyle, 2-butyl octyle, 2-pentyl nonyle, 2-hexyl décyle, 2-octyl dodécyle, 4-méthyl pentyle, 5-méthyl hexyle, 6-méthyl heptyle, 15-méthyl pendadécyle, 16-méthyl heptadécyle, 2-hexyl octyle, 2-octyl décyle ou 2-hexyl dodécyle, et n représente un nombre supérieur ou égal à un et inférieur ou égal à cinquante ; les homopolymères du N,N,N-triméthyl 3-[(2-méthyl 1-oxo 2-propènyl) amino] propanammonium, du N,N,N-triméthyl 3-[(1-oxo 2-propènyl) amino] propanammonium, du diallyl diméthylammonium, du N,N,N-triméthyl 2-[(2-méthyl 1-oxo 2-propènyl)] éthanammonium, et plus particulièrement du chlorure de N,N,N-triméthyl 3-[(2-méthyl 1-oxo 2-propènyl) amino] propanammonium (MAMPTAC^{™}), du chlorure de N,N,N-triméthyl 3-[(1-oxo 2-propènyl) amino] propanammonium (APTAC^{™}) , du chlorure de diallyl diméthylammonium (DADMAC^{™}), ou du N,N,N-triméthyl 2-[(2-méthyl 1-oxo 2-propènyl)] éthanammonium (MADQUAT^{™} ; Les copolymères branchés ou réticulés du chlorure de N,N,N-triméthyl 3-[(2-méthyl 1-oxo 2-propènyl) amino] propanammonium, du chlorure de N,N,N-triméthyl 3-[(1-oxo 2-propènyl) amino] propanammonium, du chlorure de diallyl diméthylammonium ou du N,N,N-triméthyl 2-[(2-méthyl 1-oxo 2-propènyl)] éthanammonium avec l'acrylamide, le méthacrylamide, la vinylpyrolidone, l'acrylate de (2-hydroxyéthyle) le méthacrylate de (2-hydroxyéthyle), l'acrylate de (2,3-dihydroxy propyle), le méthacrylate de (2,3-dihydroxy propyle), l'hydroxyéthylacrylamide, le N,N-diméthyl acrylamide, le N,N-diméthyl méthacrylamide, le N,N-diéthyl acrylamide, le N,N-diéthyl méthacrylamide, le N-méthyl acrylamide, le N-éthyl acrylamide, le N-propyl acrylamide, le N-isopropyl acrylamide, le N-butyl acrylamide, le N-(tert-butyl) acrylamide, le N-méthyl méthacrylamide, le N-éthyl méthacrylamide, le N-propyl méthacrylamide, le N-isopropyl méthacrylamide, le N-butyl méthacrylamide, le N-(tert-butyl)méthacrylamide, le N,N-dipropyl acrylamide ou le tris(hydroxyméthyl) acrylamido méthane (THAM) ;
- les polymères commercialisés sous les noms de marque CARBOPOL^{™}, PEMULEN^{™}, ARISTOFLEX^{™} , ARISTOFLEX ^{™}AVC, ARISTOFLEX ^{™}AVS, ARISTOFLEX ^{™}HMB, SEPIMAX^{™} Zen, SEPIMAX^{™} C, SEPIGEL^{™} 305, SEPIGEL^{™} 501, SEPIGEL^{™} 502, SIMULGEL^{™} 600, SIMULGEL^{™} EG, SIMULGEL^{™} EPG, SIMULGEL^{™} NS, SIMULGEL^{™} INS 100, SIMULGEL^{™} FL, SIMULGEL^{™} SMS 88, SIMULGEL^{™} 800, SIMULGEL^{™} A, SEPIPLUS^{™} 400, SEPIPLUS^{™} 250, SEPIPLUS^{™} S, SEPIPLUS^{™} NUDE, SEPILIFE^{™} G305, FlOCARE^{™} ET 25, FlOCARE^{™} ET 75, FlOCARE^{™} ET 26, FlOCARE^{™} ET 30, FlOCARE^{™} ET 58, FlOCARE^{™} PSD 30, VISCOLAM^{™} AT 64, VISCOLAM^{™} AT 100P, VISCOLAM^{™} AT EF, NOVEMER^{™} EC-1, NOVEMER^{™} EC-2, COSMEDIA^{™} SP, COSMEDIA^{™} ACE, SEPINOV^{™} EMT 10, SEPINOV^{™} WEO et SEPINOV^{™} P88;
- les agents épaississants ou gélifiants de type polysaccharidique non réticulés comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1 ) ; les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme arabique et de gomme de karaya, les glucosaminoglycanes.

Les agents solubilisants mis en œuvre dans le cadre de la présente invention, sont des substances ou des compositions chimiques qui permettent de solubiliser des substances hydrophobes, insolubles dans l'eau la phase aqueuse ou dans la phase hydro-alcoolique ou dans la phase hydro-glycolique, comme les substances parfumantes et aromatisantes. Comme exemples de tels agents optionnellement présents dans la formulation topique cosmétique ou pharmaceutique objet de l'utilisation ou de l'invention telles que définies ci-dessus, il y a :
- les Polysorbates, comme le Polysorbate 20, le Polysorbate 60 et le Polysorbate 80 ;
- les compositions d'alkylpolyglycosides dont la chaîne alkyle linéaire ou ramifiée comprend de 4 à 10 atomes de carbone, comme le n-butylpolyglucoside, le n-butylpolyxyloside, le n-pentylpolyglucoside, le n-pentylpolyxyloside, le n-hexylpolyglucoside, le n-hexylpolyxyloside, le n-heptylpolyglucoside, le n-heptylpolyxyloside, le n-octylpolyglucoside, le n-octylpolyxyloside, le n-nonylpolyglucoside, le n-nonylpolyxyloside, le n-décylpolyglucoside, le n-décylpolyxyloside ;
- les alcools gras éthoxylés de formule R10-(OE)n'-H, R10, représentant un radical aliphatique linéaire ou ramifié, saturé ou insaturé, comportant de 12 à 22 atomes de carbone, de préférence de 12 à 18 atomes de carbone, et encore particulièrement de 12 à 16 atomes de carbone, et n'représentant un nombre entier compris supérieur ou égal à 5 et inférieur ou égal à 200, plus particulièrement supérieur ou égal à 5 et inférieur ou égal à 100, plus particulièrement supérieur ou égal à 10 et inférieur ou égal à 100 ; par exemple le s composés de formule R10-(OE)n'-H, R10, représentant le radical dodécyle et n représentant un nombre entier supérieur ou égal à 7 et inférieur ou égal à 25 ;
- Les acides gras polyéthoxylés de formule R20-C(=O)-(OE)m', R20 représentant un radical aliphatique linéaire ou ramifié, saturé ou insaturé, comportant de 12 à 22 atomes de carbone, plus particulièrement de 12 à 18 et m' représentant un nombre entier supérieur ou égal à 10 et inférieur ou égal à 100, plus particulièrement supérieur ou égal à 15 et inférieur ou égal à 100, et encore plus particulièrement supérieur ou égal à 15 et inférieur ou égal à 50 ;
- des huiles hydrogénées et éthoxylées , et plus particulièrement celles comprenant au moins un triglycéride ou un diglycéride ou un monoglycéride comme l'huile de ricin hydrogénée et éthoxylée à 40 moles d'oxyde d'éthylène commercialisée sous l'appellation « PEG-40 hydrogenated castor oil » ;

Comme exemples d'agents émulsionnants optionnellement présents dans la formulation topique cosmétique ou pharmaceutique objet de l'utilisation ou de l'invention telles que définies ci-dessus, on peut citer ceux sélectionnés parmi les éléments du groupe constitué par les compositions d'alkylpolyglycosides et notamment les alkylpolyglucosides et le alkylpolyxylosides, les compositions d'alkylpolyglycosides et d'alcools gras, les esters de polyglycérols et notamment l'oléate de décaglycérol, l'isostéarate de décaglycérol, le monolaurate de décaglycérol, le mono-linoléate de décaglycérol, le mono-myristate de décaglycérol, les esters de polyglycérols alcoxylés, les polyhydroxystéarates de polyglycols, les polyhydroxystéarates de polyglycérols, les polyhydroxystéarates de polyglycérols alcoxylés.

Comme exemples d'huiles optionnellement présents dans la formulation topique cosmétique ou pharmaceutique objet de l'utilisation ou de l'invention telles que définies ci-dessus, on peut citer :
- Les alcanes linéaires comportant de onze à dix-neuf atomes de carbone, comme l'undécane, le dodécane, le tridécane, le tétradécane, le pentadécane, l'hexadécane, l'heptadécane, l'octadécane et le nonadécane;
- Les alcanes ramifiés, comportant de sept à quarante atomes de carbone, comme l'isododécane, l'isopentadécane, l'isohexadécane, l'isoheptadécane, l'isooctadécane, l'isononadécane ou l'isoeicosane), ou des mélanges de certains d'entre eux comme ceux cités ci-après et identifiés par leur nom INCI : C₇₋₈ isoparaffin, C₈₋₉ isoparaffin, C₉₋₁₁ isoparaffin, C₉₋₁₂ isoparaffin, C₉₋₁₃ isoparaffin, C₉₋₁₄ isoparaffin, C₉₋₁₆ isoparaffin, C₁₀₋₁₁ isoparaffin, C₁₀₋₁₂ isoparaffin, C₁₀₋₁₃ isoparaffin, C₁₁₋₁₂ isoparaffin, C₁₁₋₁₃ isoparaffin, C₁₁₋₁₄ isoparaffin, C₁₂₋₁₄ isoparaffin, C₁₂₋₂₀ isoparaffin, C₁₃₋₁₄ isoparaffin, C₁₃₋₁₆ isoparaffin ;
- Les cyclo-alcanes optionnellement substitués par un ou plusieurs radicaux alkyles linéaires ou ramifiés ;
- Les huiles blanches minérales, comme celles commercialisées sous les noms suivants : MARCOL^{™} 52, MARCOL^{™} 82, DRAKEOL^{™} 6VR, EOLANE^{™} 130 et EOLANE^{™} 150 ;
- L'hémisqualane (ou 2,6,10-trimethyl- dodécane ; numéro CAS : 3891-98-3), le squalane (ou 2,6,10,15,19,23-hexamethyltetracosane), le polyisobutène hydrogéné ou le polydécène hydrogéné ;
- Les mélanges d'alcanes comportant de 15 à 19 atomes de carbone, lesdits alcanes étant des alcanes linéaires, des alcanes ramifiés et des cyclo-alcanes, et plus particulièrement le mélange (M₁) qui comprend pour 100% de sa masse, une proportion massique en alcanes ramifiés supérieure ou égale à 90% et inférieure ou égale à 100% ; une proportion massique en alcanes linéaires supérieure ou égale à 0% et inférieure ou égale à 9%, et plus particulièrement inférieure à 5% et une proportion massique en cyclo-alcanes supérieure ou égale à 0% et inférieure ou égale à 1%, par exemple les mélanges commercialisés sous les noms EMOGREEN^{™} L15 ou EMOGREEN ^{™} L19 ;
- Les éthers d'alcool gras de formule Z1-O-Z2, dans laquelle Z1 et Z2, identiques ou différents, représentent un radical alkyle linéaire ou ramifié comportant de cinq à dix-huit atomes de carbone, par exemple les dioctyl éther, didécyl éther, didodécyl éther, dodécyl octyl éther, dihexadécyl éther, (1,3-diméthyl butyl) tétradécyl éther, (1,3-diméthyl butyl) hexadécyl éther, le bis(1,3-diméthyl butyl) éther ou le dihexyl éther ;
- Les mono-esters d'acides gras et d'alcools de formule R'1-(C=O)-O-R'2 dans laquelle R'1-(C=O) représente un radical acyle, saturé ou insaturé, linéaire ou ramifié, comportant de huit à vingt-quatre atomes de carbone et R'2 représente, indépendamment de R'1, une chaîne hydrocarbonée saturée ou insaturée, linéaire ou ramifiée comportant de un à vingt-quatre atomes de carbone, par exemple les laurate de méthyle, laurate d'éthyle, laurate de propyle, laurate d'isopropyle, laurate de butyle, laurate de 2-butyle, laurate d'hexyle, cocoate de méthyle, cocoate d'éthyle, cocoate de propyle, cocoate d'isopropyle, cocoate de butyle, cocoate de 2-butyle, cocoate d'hexyle, myristate de méthyle, myristate d'éthyle, myristate de propyle, le myristate d'isopropyle, le myristate de butyle, le myristate de 2-butyle, le myristate d'hexyle, le myristate d'octyle, le palmitate de méthyle, le palmitate d'éthyle, le palmitate de propyle, le palmitate d'isopropyle, le palmitate de butyle, le palmitate de 2-butyle, le palmitate d'hexyle, le palmitate d'octyle , l'oléate de méthyle, l' oléate d'éthyle, l'oléate de propyle, l'oléate d'isopropyle, l'oléate de butyle, l'oléate de 2-butyle, l'oléate d'hexyle, l'oléate d'octyle, le stéarate de méthyle, le stéarate d'éthyle, le stéarate de propyle, le stéarate d'isopropyle, le stéarate de butyle, le stéarate de 2-butyle, le stéarate d'hexyle, le stéarate d'octyle, l'isostéarate de méthyle, l'isostéarate d'éthyle, l'isostéarate de propyle, l'isostéarate d'isopropyle, l'isostéarate de butyle, l'isostéarate de 2-butyle, l'isostéarate d'hexyle, ou l'isostéarate d'isostéaryle ;
- Les di-esters d'acides gras et de glycérol de formule R'3-(C=O)-O-CH2-CH(OH)-CH2-O-(C=O)-R'4 et de formule R'5-(C=O)-O-CH2-CH(O-(C=O)-R'6)-CH2-OH dans lesquelles R'3-(C=O), R'4-(C=O), R'5-(C=O), R'6-(C=O), identiques ou différents, représentent un groupement acyle, saturé ou insaturé, linéaire ou ramifié, comportant de huit à vingt-quatre atomes de carbone ;
- Les tri-esters d'acides gras et de glycérol de formule R'7-(C=O)-O-CH2-CH(O-(C=O)-R"8)-CH2-O-(C=O)-R"9, dans laquelle R'7-(C=O), R'8-(C=O) et R'9-(C=O), identiques ou différents, représentent un groupement acyle, saturé ou insaturé, linéaire ou ramifié, comportant de huit à vingt-quatre atomes de carbone.
- Les huiles végétales, telles que le phytosqualane ou les huiles d'amandes douces, de coprah, de ricin, de jojoba, d'olive, de colza, d'arachide, de tournesol, de germes de blé, de germes de maïs, de soja, de coton, de luzerne, de pavot, de potiron, d'onagre, de millet, d'orge, de seigle, de carthame, de bancoulier, de passiflore, de noisette, de palme, de noyau d'abricot, de calophyllum, de sisymbrium, d'avocat, calendula ou les huiles issues de fleurs ou de légumes ;
- Les huiles végétales éthoxylées.

Dans la présente invention, on désigne par "cire", un composé ou un mélange de composés insolubles dans l'eau, se présentant sous un aspect solide à une température supérieure ou égale à 45°C. Comme exemples de cires optionnellement présents dans la formulation topique cosmétique ou pharmaceutique objet de l'utilisation ou de l'invention telles que définies ci-dessus, on peut citer les cires d'abeille, de carnauba, de candelilla, d'ouricoury, du Japon, de fibre de liège, de canne à sucre, de paraffines, de lignite, de lanoline, d'ozokérite, polyéthylène ou de silicone ; les cires végétales ou microcristallines, les alcools gras, les acides gras et les glycérides solides à température ambiante comme les beurre de karité ou de caco.

Comme exemples d'eaux thermales et minérales naturelles optionnellement présents dans la formulation topique cosmétique ou pharmaceutique objet de l'utilisation ou de l'invention telles que définies ci-dessus, on peut citer les eaux d'Avène, de Vittel, d'Uriage, de la Roche-Posay, de la Bourboule, d'Enghien-les-Bains, de Saint-Gervais-les bains, de Néris-les-Bains, d'Allevard-les-bains, de Digne, des Maizières, de Neyrac, de Lons le Saunier, de Rochefort, de Saint-Christau, des Fumades, de Tercis-les-Bains, de Bagnères-de-Bigorre, d'Eugenie-les-Bains, l'eau de Challes-les-Eaux, de Volvic, de Vais, de Vernière, d'Aix les Bains, d'Alet, des Abatilles, d'Arcens, d'Arvie, d'Asperjoc, de Badoit, de Cilaos, de Contrexéville, d'Evian, d'Hépar, de Jouvence, du Mont-Roucous, d'Ogeu, d'Orezza, de Parot, de Perrier, de Plancoët, de Quézac, de Rozana, de Saint-Alban-Les-Eaux, de Saint-Amand-les Eaux, de Saint-Georges, de Saint-Géron, de Sainte-Marguerite, de Saint-Yorre, de la Salvetat, de Teissières-lès-Bouliès, de Thonon, Treignac, de Courmayeur, de San Benedetto, de San Pellegrino ou bassin de Vichy.

Comme exemples d' agents déodorants optionnellement présents dans la formulation topique cosmétique ou pharmaceutique objet de l'utilisation ou de l'invention telles que définies ci-dessus, on peut citer les silicates alcalins, les sels de zinc comme les sulfate gluconate, chlorure ou lactate de zinc ; les sels d'ammonium quaternaires comme les sels de cétyltriméthylammonium, les sels de cétylpyridinium ; les dérivés du glycérol comme les caprate ou caprylate de glycérol, le caprate de polyglycérol, le capryloyl glycine, le 1,2 décanediol, le 1,3 propanediol, l'acide salicylique, le bicarbonate de sodium, les cyclodextrines, les zéolithes métalliques, le TRICLOSAN^{™} ; les sels d'aluminium comme les bromhydrate, les chlorhydrate, chlorure, sulfate ou lactate d'aluminium , les chlorhydrates mixtes d'aluminium et de zirconium comme les chlorhydrate, trichlorhydrate, tétrachlorhydrate, pentachlorhydrate ou octochlorhydrate d'aluminium et de zirconium ; le lactate de sodium et d'aluminium ; les complexes de chlorhydrates d'aluminium et de diols comme les complexes chlorhydrate d'aluminium-glycol ou -propylèneglycol, le complexe dichlorhydrate d'aluminium-propylène glycol, le complexe de sesquichlorhydrate d'aluminium-propylène glycol, le complexe chlorhydrate d'aluminium-polyéthylène glycol, le complexe dichlorhydrate d'aluminium-polyéthylène glycol ou le complexe sesquichlorhydrate d'aluminium-polyéthylène glycol.

Comme agents hydrotropes optionnellement présents dans la formulation topique cosmétique ou pharmaceutique objet de l'utilisation ou de l'invention telles que définies ci-dessus, on peut citer les xylènes sulfonates, les cumènes sulfonates, l'hexylpolyglucoside, le 2-éthylhexylpolyglucoside ou le n-heptylpolyglucoside.

Comme agents anti-oxydants optionnellement présents dans la formulation topique cosmétique ou pharmaceutique objet de l'utilisation ou de l'invention telles que définies ci-dessus, on peut citer l'EDTA (acide éthylenediamine tétra-acétique) et ses sels, l'acide citrique, l'acide tartarique, l'acide oxalique, le BHA (butylhydroxyanisol), le BHT (butylhydroxytoluène), les dérivés de tocophérol tels que l'acétate de tocophérol, la DISSOLVINE^{™} GL 47S ou le CONTACTICEL^{™}.

Comme exemples de filtres solaires optionnellement présents dans la formulation topique cosmétique ou pharmaceutique objet de l'utilisation ou de l'invention telles que définies ci-dessus, on peut citer tous ceux figurant dans la directive cosmétique 76/768/CEE modifiée annexe VII

Comme exemples d'agents solubilisant de filtres organiques optionnellement présents dans la formulation topique cosmétique ou pharmaceutique objet de l'utilisation ou de l'invention telles que définies ci-dessus, on peut citer le LANOL^{™} 37T, le DUB^{™} 810PGM, le DUB^{™} DIS, le DUB^{™}DIPA, le DUB^{™} DNPG ou le DUB^{™} SYNERSOL.

Comme exemple d'écran solaire minéral optionnellement présents dans la formulation topique cosmétique ou pharmaceutique objet de l'utilisation ou de l'invention telles que définies ci-dessus, on peut citer les oxydes de titane, de zinc, de cérium, de zirconium, de fer jaune, rouge ou noir, ou de chrome.

Comme exemples de solvants et de co-solvants optionnellement présents dans la formulation topique cosmétique ou pharmaceutique objet de l'utilisation ou de l'invention telles que définies ci-dessus, on peut citer le glycérol, le diglycérol, les oligomères du glycérol, l'éthylène glycol, le propylène glycol, le butylène glycol, l'hexylène glycol, le diéthylène glycol, le xylitol, l'érythritol, le sorbitol, les alcools hydrosolubles tels que l'éthanol, l'isopropanol ou le butanol, les mélanges d'eau et desdits solvants organiques, le propylène carbonate, l'acétate d'éthyle, l'alcool benzylique, le diméthylsulfoxyde (DMSO).

Comme exemples d'agents d'amélioration de la pénétration cutanée optionnellement présents dans la formulation topique cosmétique ou pharmaceutique objet de l'utilisation ou de l'invention telles que définies ci-dessus, on peut citer les glycolséthers comme l'éthylène glycol monométhyl éther, l'éthylène glycol monoéthyl éther, l'éthylène glycol monopropyl éther, l'éthylène glycol monoisopropyl éther, l'éthylène glycol monobutyl éther, l'éthylene glycol monophényl éther, l'éthylène glycol monobenzyl éther, le diéthylène glycol monométhyl éther, le diéthylène glycol monoéthyl éther et le diéthylène glycol mono-n-butyl éther, le diéthylène glycol monoéthyléther (ou Transcutol-P), les acides gras comme l'acide oléique, les esters de glycérol d'acide gras comme le béhénate de glycérol, le palmitostéarate de glycérol, le béhénoyl macroglycérides, le polyoxyéthylène-2-stéaryl éther, le polyoxyéthylène-2-oléyl éthers, des terpènes comme le D-Limonène, des huiles essentielles comme l'huile essentielle d'eucalyptus.

Comme exemples d'agents stabilisants optionnellement présents dans la formulation topique cosmétique ou pharmaceutique objet de l'utilisation ou de l'invention telles que définies ci-dessus, on peut citer les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples de principes actifs optionnellement présents dans la formulation topique cosmétique ou pharmaceutique objet de l'utilisation ou de l'invention telles que définies ci-dessus, on peut citer :
- les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ;
- les composés montrant une action éclaircissante ou dépigmentante de la peau comme le ω-undecelynoyl phénylalanine commercialisé sous l'appellation SEPIWHITE^{™}MSH, le SEPICALM^{™}VG, le mono ester ou le diester de glycérol du ω-undecelynoyl phénylalanine, les ω-undecelynoyl dipeptides, l'arbutine, l'acide kojique, l'hydroquinone ;
- les composés montrant une action apaisante notamment le SEPICALM^{™} S, l'allantoïne et le bisabolol;
- les agents analgésiques ou anti-inflammatoires comme l'acétaminophène, l'aspirine, l'acide salicylique, les salicylate de méthyle, de choline ou de glycol, le 1-menthol, le camphre, l'acide méfénamique, l'acide fluphénamique, l'indométhacine, l'acide protizidique, le fentiazac, le tolmetin, le phénylbutazone, l'oxyphenbutazone, le clofézone, le pentazocine, le mépirizole, l'hydrocortisone, la cortisone, la dexaméthasone, la fluocinolone, la triamcinolone, la médrysone, la prednisolone, la flurandrénolide, le prednisone, l'halcinonide, le méthylprednisolone, le fludrocortisone, la corticostérone, la paraméthasone, la bétaméthasone ; les acide (hétéro)aryl acétique ou 2-(hétéro)aryl propionique comme le diclofénac, l'acide tiaprofènique, l'alminoprofène, l'étodolac, le flurbiprofène, l'ibuprofène, le kétoprofène ou le naproxène.
- les agents antiseptiques comme la cétrimide, la povidone-iodine, la chlorhexidine, la viodine, le chlorure de benzalkonium, l'acide benzoïque, le nitrofurazone, le peroxyde de benzoyle, le peroxyde d'hydrogène, l'hexachlorophène, le phénol, le résorcinol ou le chlorure de cétylpyridinium ;
- les agents insecticides comme le trichlorfone, le triflumérone, le fenthion, le bendiocarbe, la cyromazine, le dislubenzurone, le dicyclanile, le fluazurone, l'amitraze, la deltaméthrine, la cyperméthrine, le chlorfenbinphose, la fluméthrine, la ivermectine, l'abermectine, l'avermectine, la doramectine, la moxidectine, la zeticyperméthrine, la diazinone, la spinosade, l'imidaclopride, le nitenpyrane, le pyriproxysene, le sipronil, le cythioate, la lufénurone, la selamectine, la milbemycine oxime, le chlorpyrifose, le coumaphose, le propetamphose, Valpha-cyperméthrine, l'highciscyperméthrine, ivermectine, la diflubenzurone, le cyclodiène, le carbamate and ou la benzoyl urée.
- les agents antimicrobiens comme les sulfonamides, la néomycine, le tobramycine, le gentamycine, l'amikacine, le kanamycine, le spectinomycine, le paromomycine, le netilmicine, les polypeptides, les céphalosporines ou les oxazolidinones comme la ciprofloxacine, la levofloxacine ou l'ofloxacine ;
- les composés montrant une action hydratante comme l'urée, les hydroxy urées, le glycérol, les polyglycérols, le glycérolglucoside, le diglycérolglucoside, les polyglycérylglucosides ou le xylitylglucoside ;
- les extraits végétaux riches en polyphénols comme les extraits de raisin, les extraits de pin, les extraits de vin, les extraits d'olives ;
- les composés montrant une action amincissante ou lipolytique comme la caféine ou ses dérivés, l'ADIPOSLIM^{™}, l'ADIPOLESS^{™}, la fucoxanthine ;
- les protéines N-acylées, les peptides N-acylés comme le MATRIXIL^{™}, les acides aminés N-acylés, les hydrolysâts partiels de protéines N-acylés, les acides aminés, les peptides, les hydrolysâts totaux de protéines,
- Les extraits de végétaux, comme les extraits de soja, par exemple la Raffermine^{™}, les extraits de blé par exemple la TENSINE^{™} ou la GLIADINE^{™}, les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ;
- les cires essentielles ;
- les extraits bactériens ;
- les céramides ;
- les phospholipides ;
- les composés montrant une action antimicrobienne ou une action purifiante, comme le LIPACIDE^{™} C8G, le LIPACIDE^{™} UG, le SEPICONTROL^{™} A5 ; l'OCTOPIROX^{™} ou le SENSIVA^{™} SC50 ;
- Les composés montrant une propriété énergisante ou stimulante comme le PHYSIOGENYL^{™},
- le panthénol et ses dérivés comme le SEPICAP^{™} MP ;
- Les actifs anti-âge comme le SEPILIFT^{™} DPHP, le LIPACIDE^{™} PVB, le SEPIVINOL^{™}, le SEPIVITAL^{™}, le MANOLIVA^{™}, le PHYTO-AGE^{™}, le TIMECODE^{™} ; le SURVICODE^{™} ;
- Les actifs anti-photo vieillissement, les actifs protecteurs de l'intégrité de la jonction dermo-épidermique, les actifs augmentant la synthèse des composants de la matrice extracel-lulaire comme le collagène, les élastines, les glycosaminoglycanes ;
- Les actifs agissant favorablement sur la communication cellulaire chimique comme les cytokines ou physiques comme les intégrines ;
- Les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ;
- Les actifs améliorant la microcirculation cutanée, par exemple les veinotoniques ;
- Les actifs drainants, les actifs à visée décongestionnante comme les extraits de ginko biloba, de lierre, de marron d'inde, de bambou, de ruscus, de petit houx, de centalla asiatica, de fucus, de romarin, de saule ;
- Les agents destinés au traitement des cheveux ou des poils, par exemple des agents protecteurs des mélanocytes du follicule pileux, les agents mimétiques de l'activité de la DO-PAchrome tautomérase, les molécules synthétiques mimétiques de la SOD par exemples les complexes de manganèse, des composés antioxydants par exemple les dérivés de cyclodextrine, des composés silicés dérivés d'acide ascorbique, de la pyrrolidone carboxylate de lysine ou d'arginine, des associations de mono- et diester d'acide cinnamique et de vitamine C ;
- les agents de bronzage ou de brunissement de la peau, par exemple la dihydroxyacétone (DHA), l'érythrulose, l'aldéhyde mésotartrique, le glutaraldéhyde, le glycéraldéhyde, l'alloxane, la ninhydrine, les extraits végétaux par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida ;
- les agents connus pour leur action de facilitation ou d'accélération du bronzage ou du brunissement de la peau humaine, ou pour leur action de coloration de la peau humaine, par exemple les caraténoïdes ( et plus particulièrement le beta carotène et le gamma carotène), les produits commercialisés sous le nom de marque « Carrot oil » et « SunTan Accelerator^{™} »,v« Zymo Tan Complex », « MelanoBronze^{™} », « Monk's pepper extract », « Unipertan VEG-24/242/2002, « », « Try-Excell^{™} », «Actibronze^{™} », « Tyrostan^{™} », « Tyrosinol », « Insta-Bronze^{™} », « Tyrosilane », « Exymol » »Bronzing SF Peptide powder », « Melitane », « Melatimes Solutions^{™} », « Tanositol^{™} », « Thalitan^{™} , « Phycosaccharide^{™} AG », « Melactiva^{™} » et « Biotanning^{™}».
- Comme substances parfumantes ou aromatisantes optionnellement présentes dans la formulation topique cosmétique ou pharmaceutique objet de l'utilisation ou de l'invention telles que définies ci-dessus, on peut citer celles extraites de fleurs comme la rose, le jasmin, la tubéreuse, la champaca, le mimosa, l'œillet, l'osmanthe, la narcisse, la lavande le gardénia ou les fleurs de frangipanier, d'Ylang-ylang, de lotus, d'acacia, d'oranger doux, d'oranger amer ou néroli ; celles extraites de feuilles, de mousse, d'écorce, de résine ou de bourgeons comme les bourgeons de cassis ; les mousses de chêne, de hêtre ou le lichen ; les feuilles d'acacia, de basilic, de valériane, de gentiane, de violette, de géranium, de labdanum, de romarin, de patchouli ou de verveine ; les écorces de cannelle, de frêne, de cassia ou de cascarille ; les bois de santal, de cèdre, de palissandre, d'aloès, de bouleau, de gaïac ; les baumes du Pérou ou du tolu ; la résine de Benjoin, la myrrhe, la résine de labdanum, la résine d'élémi, l'oliban, l'opoponax, le guggul, celles extraites d'aiguilles et de branches de pin ou d'épicéa ou de sapin ; celles extraites de l'estragon, du lemon grass, de la sauge ou du thym ; celles extraites de gousses, de fèves ou de baies comme la fève de tonka, les gousses de vanille, la cardamome, la coriandre, la badiane, l'amande amère, le cumin, les clous de girofle, les baies de genièvre ; oucelles extraites des agrumes comme le citron, l'orange dont la linette et la bergamote, la mandarine ; celles extraites des racines comme les racines d'angélique, de céleri, de cardamone, d'iris, d'accore, de cactus ou de vétyver.

Comme substances parfumantes ou aromatisantes optionnellement présentes dans la formulation topique cosmétique ou pharmaceutique objet de l'utilisation ou de l'invention telles que définies ci-dessus, on peut aussi citer celles d'achillée millefeuille, d'accore calamus, d'ail, d'ajowan, d'amyris, d'aneth, d'anis, d'angélique, d'arbre à thé, de basilic, de bay saint thomas, de benjoin de bergamote, de bois de Gaïac, de bois de Hô, de bois de rose, de bois de santal, de bois de siam, de bouleau noir, de camomille, de camphrier, de cannelle, de cardamone, de carotte, de carvi, de cèdre, de céleri, de christe marine, de ciste, de citron, de citronnelle, de clémentine, l'huile de combawa, de copahu, de coriandre, de cryptomeria, de cumin, de curcuma, de cyprès, d'encens, l'huile d'épinette, d'estragon, de fenouil, de fragonia, de galbanum, de gaulthérie (wintergreen), de genévrier, de géranium, de gingembre, de clou ou de feuille de girofle, d'helichryse, d'hysope, d'iary, d'inule, de katrafay, de Khella, de kunzea, de lavande, de lavandin, de mandarine, de niaouli, de menthe poivrée, d'orange, de pamplemousse, de romarin, de thym, d'Ylang Ylang, de ravintsara, de sauge, de Cabreuva, de Lemongrass, de Palmarosa, de millepertuis, de jasmin, de camomille, de mélisse, de pin, de gingembre, de persil, d'armoise, de chanvre, de houblon, ou encore de serpolet.

Comme substances parfumantes ou aromatisantes optionnellement présentes dans la formulation topique cosmétique ou pharmaceutique objet de l'utilisation ou de l'invention telles que définies ci-dessus, on peut encore citer le musc, le castoreum, la civette, l'ambre gris, l'absolue de cire d'abeille, l'hyraceum.

Comme composés chimiques substances parfumantes ou aromatisantes synthétiques optionnellement présentes dans la formulation topique cosmétique ou pharmaceutique objet de l'utilisation ou de l'invention telles que définies ci-dessus, on peut citer :
- Des hydrocarbures terpéniques (mono et sesquiterpènes) tels que l'alpha ou le béta myrcène, le limonène, l'alpha ou le béta pinène, le camphène, le cadinène, le cedrène, le farnesène, le caryophyllène, le chamazulène, 1,1-diméthoxy-2,2,5-triméthyl-4-hexène, le curcumène, le crythmène, les himachelènes, le limonène ou le paracymène ; les oxydes de rose ou de tétranorlabdane ; les terpinènes, les terpinolènes ou les vetivènes ;
- Des esters tels que les acétates de benzyle, de bornyle, de citronellyle, de cédryle, de dihydromyrcényle, de diméthyl-benzylcarbinyle, d'éthyle, de farnésyle, de fenchyle, d'hexyle, de géranyle, d'isobutyle, d'isononyle, d'isopentyle, d'isobornyle, d'isopulégyle, de linalyle, de menthyle, de méthyl phényl carbinyle, de néryle, de nonyle, de 2-(tert-butyl) cyclohexyle, de phényléthyle, de 4-(tert-butyl) cyclohexyle, de prényle, de styrallyle, de terpènyle ou de vetyvéryle ; l'anthralinate de methyle, les benzoates de benzyle, d'isobutyle ou de linalyle, la coumarine, les butanoates d'éthyle, de benzyle ou d'isoamyle, les butyrates de benzyle, d'éthyle, d'isoamyle, ou de lynalyle ; les cinnamates de butyle, d'allyle ou d'éthyle, les formiates de benzyle, de citronellyle, d'hédione, de geranyle, de méthyle ; le glycinate d'éthylméthylphényle, le glycolate d'allyl-amyle, l'heptanoate d'allyle, l'isobutyrate de phénoxyéthyle, l'isobutyrate de cis-3-hexényle, le méthacrylate d'isoamyle, le naphtolate d'ethyle, le néopentanoate d'hexyle, les propionates d'amyle, d'alkylcyclohexyle, d'allylcyclohexane, de lynalyle, de styralyle ou de citronellyle ; les salicylates de méthyle, de benzyle ou d'éthyle ou le tiglate d'hexyle ;

- Des alcools et des phénols tels que l'alcool benzoïque, l'alpha terpinéol, l'anéthol, le carotol, le chavicol, l'estragol , le cinéol, l'alcool cinnamique, le citronellol, le p-crésol, l'alcool cumique, le 3,7-diméthyl-1-octanol, le diméthyle benzyle carbinol, l'alcool fenchylique, le l'eucalyptol, le farnesol, l'eugénol, l'alcool isononylique, l'isoeugénol, le gaïacol, le géraniol, le globutol, le linanol, le menthol, le dihydromyrcénol, le nérolidol, le nérol, l'alcool phenyl éthylique, le safrol, l'isosafrol, le phytol, l'iso phytol, le terpineol, le tétrahydrolinalol, le tétrahydromyrcénol, le thymol, le vétivérol ou l'undécavertol ;
- Des aldéhydes tels que les aldéhydes phényl acétique, salicylique, anisique, caprylique, cinnamique ou hexyl cinnamique ; le bourgeonal, le citral, le citronellal, l'hydroxy-citronellal, le citronellyloxyacétaldéhyde, le cyclamènaldéhyde, le cuminaldéhyde, le cyclal, le 2,4-diméthyl-3-cyclohexène-1-carboxaldéhyde, le dodécanal, l'éthanal, l'octanal, le décanal, les géranials, l'hélional, les lactones telles que les gamma-undécalactones, le lilial, le méthyl n-nonyl acétaldéhyde, le méthyl octyl acétaldéhyde, l'undécanal ou la vanilline ;
- Des cétones tels que la benzyl acétone, la 7-méthyl-2H-benzo-1,5-dioxepin-3(4H)-one, la carvone, le camphre, la civéttone, les damascones, les damascénones, l'éthyl-amyl-cétone, l'éthyl-hexyl-cétone, la géranyl cétone, la jasmone, les irones, le 3-hydroxy-2-methyl-4H-pyran-4-one, l'éthyl maltol, la menthone, l'isomenthone, la muscone, la méthyl hepténone, les ionones comme la méthyl-ionone, la 4-méthyl acétophénone, la méthylpentylcétone, la méthylheptylcétone, la méthylhexylcétone, l'alpha-isométhylionone, ou la méthylcédrylcétone ;
- des éthers tels que l'anéthol, le benzyléthléther, le cédryl methyl ether, le p-crésyl méthyl éther ;
- des muscs artificiels issus de différents composés nitrés, les musc ambrette, les musc cétones, les musc xylène, les musc macrocycliques,
- des nitriles tels que les triméthyl-3 , 5, 7-octane (ène) nitriles et leurs dérivés alpha-substitués, le citronellyl nitrile, le citronitrile, le géranyl-nitrile.

Les exemples suivants illustrent l'invention sans toutefois la modifier.

Préparation de xanthane réticulé par du STMP dans un mélange eau-éthanol (selon l'invention). Le procédé de synthèse comprend les étapes suivantes :
- Etape A) : Chargement dans un réacteur double enveloppe en verre d'un litre sous agitation mécanique de 193 g d'un mélange hydroalcoolique d'éthanol 95% et d'eau dans un ratio massique 62/38 et de 30 g de xanthane,
- Etape c) : Ajustement, à 25°C, du pH du mélange préparé à l'étape A) à une valeur de pH égale à 12,5 à l'aide d'une solution aqueuse tétramolaire de soude,
- Etape d) : Addition dans le mélange issu de l'étape c) et chauffé à une température de 50°C de 0,018 g de STMP (soit 0,06% massique par rapport au xanthane) ; maintien sous agitation à cette température pendant deux heures, puis refroidissement jusqu'à 25°C,
- Etape e) : Ajustement du pH du milieu réactionnel à 7 à l'aide d'une solution aqueuse pentamolaire d'acide chlorhydrique,
- Etape f) : Filtration gravitaire du milieu réactionnel sur filtre papier (filtration moyenne de 4µm à 7µm),
- Etape g) : Séchage du produit récupéré à l'étape f) entre 16 et 20 heures dans une étuve thermostatée à 50°C sous vide ; on obtient ainsi 24 g de polysaccharide (P1) sous forme de poudre blanche avec un rendement égal à 80% (par rapport au xanthane introduit).

Préparation de xanthane réticulé par du STMP dans un mélange Eau-Éthanol (comparatif). Le même procédé que celui décrit au paragraphe 061 précédent a été mis en œuvre en ajustant le pH de l'étape b) à 7,0 au lieu de 12,5. 24 g de polysaccharide (P1') ont été isolés sous forme de poudre blanche avec un rendement égal à 80% (par rapport au xanthane introduit).

Préparation de gomme xanthane réticulée par du STMP dans un mélange eau-éthanol (selon l'invention). Le procédé de synthèse comprend les étapes suivantes :
- Etape A) : Chargement dans un réacteur double enveloppe en verre d'un litre sous agitation mécanique de 200 g d'un mélange hydroalcoolique d'éthanol 95% et d'eau dans un ratio massique 75/25 et de 60 g de gomme xanthane,
- Etape c) : Ajustement, à 20°C, du pH du mélange préparé à l'étape A) à une valeur de pH égale à 12,0 à l'aide d'une solution aqueuse tétramolaire de soude,
- Etape d) : Addition dans le mélange issu de l'étape c) et chauffé à une température de 35°C de 0,69 g de STMP (soit 1,15% massique par rapport au xanthane) ; maintien sous agitation à cette température pendant une heure, puis refroidissement jusqu'à 20°C,
- Etape e) : Ajustement du pH du milieu réactionnel à 7 à l'aide d'une solution aqueuse pentamolaire d'acide chlorhydrique,
- Etape f) : Filtration gravitaire du milieu réactionnel sur filtre papier (filtration moyenne de 4 à 7µm),
- Etape g) : Séchage du produit récupéré à l'étape f) entre 16 et 20 h dans une étuve à 50°C sous vide ; on obtient ainsi le polysaccharide (P2) sous forme de poudre blanche.

Préparation de xanthane lipophilisé et réticulée par du STMP dans un mélange eau -éthanol (selon l'invention). Le même procédé que celui décrit au paragraphe 061 précédent a été mis en œuvre en remplaçant la xanthane par une gomme de xanthane modifiée chimiquement, estérifiée par de l'acide dodécanoïque. 26 g de polysaccharide (P3) ont été isolés sous forme de poudre blanche avec un rendement égal à 86.7% (par rapport au xanthane lipophilisé en C-12 introduit).

Préparation de gomme de xanthane réticulée par du STMP dans un mélange eau-isopropanol (selon l'invention). Le même procédé que celui décrit au paragraphe 061 précédent a été mis en œuvre en remplaçant l'éthanol par l'isopropanol. 24,5 g de polysaccharide (P4) ont été isolés sous forme de poudre blanche avec un rendement égal à 81,7% (par rapport au xanthane introduit).

Préparation de gomme de konjac réticulée par du STMP dans un mélange eau-éthanol (selon l'invention). Le même procédé que celui décrit au paragraphe 063 précédent a été mis en œuvre en remplaçant le xanthane par la gomme de konjac. Le polysaccharide (P5) a été isolé sous forme de poudre blanche.

Préparation de gomme de guar réticulée par du STMP dans un mélange eau-éthanol (selon l'invention). Le même procédé que celui décrit au paragraphe 063 précédent a été mis en œuvre en remplaçant le xanthane par la gomme de guar. 54,3 g de polysaccharide (P6) ont été isolés sous forme de poudre blanche avec un rendement égal à 90,5% (par rapport à la gomme de guar introduite).

Préparation de kappa-carraghénane (k-carraghénane) réticulé par du STMP dans un mélange eau-éthanol (selon l'invention). Le même procédé que celui décrit au paragraphe 063 précédent a été mis en œuvre en remplaçant le xanthane par le k-carraghénane et en chauffer le milieu obtenu à l'étape A) à 80°C pendant une heure. 52,5 g de polysaccharide (P7) ont été isolés sous forme de poudre blanche avec un rendement égal à 87,5% (par rapport au k-carraghénane introduit).

Préparation d'iota-carraghénane (i-carraghénane) réticulé par du STMP dans un mélange eau-éthanol (selon l'invention). Le même procédé que celui décrit au paragraphe 068 précédent a été mis en œuvre en remplaçant le k-carraghénane par le i-carraghénane. 52,8 g de polysaccharide (P8) ont été isolés sous forme de poudre blanche avec un rendement égal à 88% (par rapport au i-carraghénane introduit).

Préparation d'i-carraghénane réticulée par du STMP dans un mélange eau-éthanol et en présence de sulfate de calcium (selon l'invention). Le même procédé que celui décrit au paragraphe 069 précédent a été mis en œuvre en introduisant à l'étape A) 0,3g de sulfate de calcium. Le polysaccharide (P9) a été isolé sous forme de poudre blanche.

Préparation de k-carraghénane réticulée par du STMP dans un mélange eau-éthanol et en présence de sulfate de calcium (selon l'invention).Le même procédé que celui décrit au paragraphe 070 précédent a été mis en œuvre en remplaçant la i-carraghénane par la k-carraghénane. 53,3 g de polysaccharide (P10) ont été isolés sous forme de poudre blanche avec un rendement égal à 88,8% (par rapport au k-carraghénane introduit).

Préparation d'un mélange de xanthane - i-carraghénane réticulé par du STMP dans un mélange eau - éthanol (selon l'invention). Le même procédé que celui décrit au paragraphe 069 précédent a été mis en œuvre en ajoutant 54 g de xanthane au niveau de l'étape A) (ratio massique xanthane - i-carraghénane = 9,0). 54,7 g de polysaccharide (P11) ont été isolés sous forme de poudre blanche avec un rendement égal à 91,2% (par rapport au mélange xanthane - i-carraghénane introduit).

Préparation d'un mélange de xanthane - i-carraghénane réticulé par du STMP dans un mélange eau-éthanol (selon l'invention). Le même procédé que celui décrit au paragraphe 072 précédent a été mis en œuvre en inversant le ratio massique xanthane - i-carraghénane. Pour ceci, 6 g de xanthane et 54 g de i-carraghénane ont été utilisés (ratio massique xanthane - i-carraghénane = 0,1). 54,4 g de polysaccharide (P12) ont été isolés sous forme de poudre blanche avec un rendement égal à 90,7% (par rapport mélange xanthane - i-carraghénane introduit).

Préparation d'un mélange de xanthane - k-carraghénane réticulé par du STMP dans un mélange eau - éthanol (selon l'invention). Le même procédé que celui décrit au paragraphe 072 précédent a été mis en œuvre en remplaçant le i-carraghénane par le k-carraghénane (ratio massique xanthane / k-carraghénane = 9,0). 53,5 g de polysaccharide (P13) ont été isolés sous forme de poudre blanche avec un rendement égal à 89,2% (par rapport au mélange xanthane - k-carraghénane introduit).

Préparation d'un mélange de xanthane - k-carraghénane réticulé par du STMP dans un mélange eau - éthanol (selon l'invention). Le même procédé que celui décrit au paragraphe 073 précédent a été mis en œuvre en inversant le ratio massique xanthane - k-carraghénane. Pour ceci, 6 g de xanthane et 54 g de k-carraghénane ont été utilisés (ratio massique xanthane - k-carraghénane = 0,1). 54,3 g de polysaccharide (P14) ont été isolés sous forme de poudre blanche avec un rendement égal à 90,5% (par rapport au mélange xanthane - k-carraghénane introduit).

Préparation des gels aqueux - Evaluation des propriétés épaississantes des polymères préparés. Les propriétés épaississantes des polysaccharides réticulés par le STMP selon l'invention Polysaccharide (P1) à Polysaccharide (P14) ont été évaluées et comparées à celles des polysaccharides correspondants non réticulés. La même caractérisation a été réalisée sur le polysaccharide (P1') dont l'étape de réticulation a été menée à un faible pH hors invention.
- Préparation de gels aqueux : La méthode de réalisation des gels aqueux consiste à introduire dans un bécher de 2 litres, la quantité d'eau nécessaire à la préparation d'un gel de 800 g soit 792 g d'eau dans le cas d'un gel contenant 1% massique de produit réticulé. On place vers le fond du bécher une hélice d'agitateur mécanique connectée à un moteur. L'agitation est démarrée et la quantité nécessaire de polysaccharide réticulé est introduite dans le bécher sous agitation. L'agitation crée un vortex qui disparaît lorsque le polysaccharide s'hydrate et forme un gel. Dans le cas particulier des polysaccharides qui développent leur viscosité sous un effet thermique, un chauffage du gel entre 50 et 100°C pour aider à la solubilisation peut être nécessaire. Une partie du gel formé est conservée pour mesure de la viscosité. L'autre partie est utilisée pour fabriquer un gel contenant 1% de polysaccharide + 0,5% de chlorure de sodium. Pour cela 398 g de gel sont placés sous agitation à l'aide d'une hélice d'agitateur mécanique connectée à un moteur, 2 g de chlorure de sodium sont ensuite ajoutés. L'agitation est maintenue jusqu'à solubilisation totale du et obtention d'un gel homogène. Les gels ainsi préparés sont évalués trois heures puis vingt-quatre heures après préparation. La viscosité du gel est mesurée à l'aide d'un viscosimètre Brookfield RVT (vitesse 5, Mobile adapté à la viscosité) ou Brookfield LVT (vitesse 6). Les résultats sont rassemblés dans les tableaux 1 à 3 suivants.

**Tableau 1**

| | Gel à 1% PS | | Gel à 2% PS | |
|---|---|---|---|---|
| | Viscosité en mPa.s | | Viscosité en mPa.s | |
| | Gain de viscosité en % *** | | Gain de viscosité en % *** | |
| Polysaccharide (PS)↓ | | | | |
| PS non réticulé↓ | Sans NaCl | 0,5% NaCl | Sans NaCl | 0,5% NaCl |
| Xanthane | 3.140 | 12.650 | 11.600 | 40.200 |
| Xanthane C-12 | 32.000 | 5.000 | nd | nd |
| Gomme de Konjac | 26.200 | 24.800 | nd | nd |
| Gomme de Guar | 12.000 | 11.800 | nd | nd |
| k-Carraghénane | 10.080 | 39.400 | nd | nd / |
| i-Carraghénane | nd | Nd | 45.600 | 45.200 |
| 1-Carraghénane + CaSO₄ | 13.400 | 10.000 | nd | nd |
| k-Carraghénane + CaSO₄ | 60.600 | 33.200 | nd | nd |
| Xanthane - i-Carraghénane (9,0) | nd | nd | 5.600 | 4.0600 |
| Xanthane - i-Carraghénane (0,1) | nd | nd | 11.400 | 11.3600 |

**Tableau 2**

| | Gel à 1% PS | | Gel à 2% PS | |
|---|---|---|---|---|
| | Viscosité en en mPa.s | | Viscosité en mPa.s | |
| | Gain de viscosité en %*** | | Gain de viscosité en %*** | |
| PS non réticulé ↓ | Sans NaCl | 0,5% NaCl | Sans NaCl | 0,5% NaCl |
| Xanthane : k-Carraghénane (9,0) | nd | nd | 8.400 | 50.000 |
| Xanthane - i-Carraghénane (0,1) | nd | nd | 11.400 | 11.3600 |
| Xanthane / κ-Carraghénane (9,0) | nd | nd | 8.400 | 50.000 |
| PS réticulé ↓ | Sans NaCl | 0,5% NaCl | Sans NaCl | 0,5% NaCl |
| Polysaccharide (P1) | 32.500 (+*935,0%*) | 13.740 (+*8,6%*) | nd | nd |
| Polysaccharide (P1') | 4.400 (+ *40,1%*) | 16.100 (+*27,3%*) | nd | nd |
| Polysaccharide (P2) | 12.000 (+*282,2%*) | 18.600 (+*47,0%*) | 29.800 *(+ 156,9%*) | 51.800 (+ 28,9%) |
| Polysaccharide (P3) | 19.000 (*+505,1%*) | 4.110 (-*67,5%*) | nd | nd |
| Polysaccharide (P4) | 23.000 (*+632,5%*) | 16.100 (+*27,3%*) | nd | nd |
| Polysaccharide (P5) | 59.600 (*+127,5%*) | 40.000 (+*61,3%*) | nd | nd |
| Polysaccharide (P6) | 12.400 (+*3,3%*) | 12.200 (+*3,4%*) | nd | nd |

**Tableau 3**

| | Gel à 1% PS | | Gel à 2% PS | |
|---|---|---|---|---|
| | Viscosité en mPa.s | | Viscosité en mPa.s | |
| | Gain de viscosité en %*** | | Gain de viscosité en %*** | |
| (PS) réticulé ↓ | Sans NaCl | 0,5% NaCl | Sans NaCl | 0,5% NaCl |
| Polysaccharide (P7) | 19.800 | 59.800 | nd | nd |
| Polysaccharide (P8) | nd | nd | 83200 (*+82,5%*) | 62800 (+*38,9%*) |
| Polysaccharide (P9) | 24.200 (*+80,6%*) | 16.200 (+*62,0%*) | nd | nd |
| Polysaccharide (P10) | 67.600 (+*11,6%*) | 103.400 (*+211,4%*) | nd | nd |
| Polysaccharide (P11) | nd | nd | 16.400 (+*192,9%*) | 46.200 (*+13,8%*) |
| Polysaccharide (P12) | nd | nd | 22.200 (+*94,7*) | 118.600 (+*4,4*) |
| Polysaccharide (P13) | nd | nd | 26000 (*+209,5%*) | 41.600 (-*16,8*) |
| Polysaccharide (P14) | nd | nd | 61800 (*+11,9%*) | 56.600 (-*62,1%*) |

| | | | | |
|---|---|---|---|---|
| Nd : non déterminé *** Gain en viscosité = [(Viscosité (PX) - Viscosité du polysaccharide correspondant non réticulé) / Viscosité du polysaccharide correspondant réticulé] x 100. | | | | |

### Conclusions

- Le bon déroulement des réactions de réticulation suivies de la précipitation du polysaccharide réticulé isolé sous forme de poudre (~ 100% de polymère) a été mis en évidence.
- Dans la majorité des cas, la réticulation des polysaccharides par du STMP permet d'augmenter la viscosité des gels à 1 ou 2% de polysaccharides réticulés en présence ou non de NaCl. Ces augmentations relatives par rapport aux mêmes polysaccharides non réticulés varient entre 3,3% (*cf* (P6)) et 935% (*cf* (P1)). Dans un nombre de cas limité, une diminution est observée (*cf* (P3), (P13) et (P14)).
- L'utilisation d'éthanol ou d'isopropanol en tant que cosolvant de réticulation permet d'atteindre des gains comparables en termes de viscosité de gel (*cf* (P1) et( P4)).
- Les exemples (P1) et P1') permettent d'apprécier l'influence du pH lors de l'étape de réticulation (respectivement 12,5 et 7,0).
- Le procédé de réticulation selon l'invention a été validé à partir de polysaccharides seuls et de mélanges de polysaccharides.

Exemples de formulation topiques cosmétiques selon l'invention

**Tableau 4**

| Formulation moussante aux AHA | |
|---|---|
| Ingrédients | Proportions massiques |
| Eau déminéralisée | qsp 100,0% |
| Glycérine | 2,0% |
| Polysaccharide (P1) | 1,0% |
| ORAMIX^{™} CG110 | 5,0% |
| Lauryl éther Sulfate de sodium 28% | 6,0% |
| Acide glycolique 70% | 5,0% |
| EUXYL^{™} K712 | 1,0% |
| Triéthanolamine | qs pH = 4 |

| Aspect visuel de la formulation moussante | |
|---|---|
| A 25°C | Homogène |
| Après 3 mois 25°C | Homogène |
| Après 3 mois 45°C | Homogène |
| pH mesuré après 7 jours à 25°C | 4,1 |
| Viscosité mesurée après 7 jours à 25°C | 9.200 mPa.s |

**Tableau 5**

| Formulation de protection solaire | |
|---|---|
| Ingrédients | Proportions massiques |
| Eau déminéralisée | qsp 100,00% |
| Polysaccharide (P1) | 0,50% |
| FLUIDIFEEL^{™} EASY | 3,00% |
| LANOL^{™} 37T | 8,00% |
| EMOGREEN^{™} L19 | 4,00% |
| NEO HELIOPAN^{™} OS | 5,00% |
| NEO HELIOPAN^{™} BMT | 4,00% |
| NEO HELIOPAN^{™} HMS | 5,00% |
| DL alpha tocophérol | 0,05% |
| EUXYL^{™} K903 | 1,00% |
| CONTACTICEL^{™} | 2,00% |
| EPHEMER^{™} | 2,00% |
| TINOSORB^{™} M | 5,00% |
| Acide citrique 25% | 0,04% |

| Aspect visuel de la formulation de protection solaire | |
|---|---|
| A 25°C | Liquide jaune homogène |
| Après 3 Mois à 25°C | Homogène |
| Après 3 Mois à 45°C | Homogène |
| pH mesuré après 7 jours à 25°C | 5,5 |
| Viscosité mesurée après 7 jours à 25°C | 6.200 mPa.s |

**Tableau 6**

| Formulation de protection solaire SPF 50 | |
|---|---|
| Ingrédients | Proportions massiques |
| Eau déminéralisée | qsp 100,0% |
| Glycérine | 3,0% |
| Polysaccharide (P1) | 0,5% |
| NEO HELIOPAN^{™} HYDRO | 1,5% |
| Triéthanolamine 50% | 1,7% |
| SENSANOV^{™} WR | 3,0% |
| NEO HELIOPAN^{™} OS | 5,0% |
| NEO HELIOPAN^{™} BMT | 6,0% |
| LANOL^{™} 37T | 7,0% |
| DUB^{™} SSIC | 5,0% |
| A15-TiO2-SX-NJE8 | 5,0% |
| DL alpha tocophérol | 0,2% |
| EUXYL^{™} PE9010 | 0,5% |
| SENSIVA^{™} PA40 | 0,5% |

| Aspect visuel de la formulation de protection solaire | |
|---|---|
| A 25°C | Jaune et homogène |
| Après 3 mois à 25°C | Homogène |
| Après 3 mois à 45°C | Homogène |
| Viscosité mesurée après 7 jours à 25°C | 58.000 mPa.s |
| pH mesuré après 7 jours à 25°C | 6,1 |

**Tableau 7**

| Emulsions de type huile dans eau | | | |
|---|---|---|---|
| Ingrédients | Proportions massiques | | |
| MONTANOV^{™} 68 | 1,0% | 1,0% | 1,0% |
| TRIGLYCERIDES 5545 | 20,0% | 20,0% | 20,0% |
| Eau déminéralisée | qsp 100,0% | | |
| Polysaccharide (P1) | 0,3% | 0,5% | 1,0% |
| EUXYL^{™} PE9010 | 1,0% | 1,0% | 1,0% |

| Aspect visuel des émulsion de type huile-dans-eau | | | |
|---|---|---|---|
| A 25°C | Homogène, liquide, blanc, lisse | | Hétérogène, liquide, blanc, grumeleux |
| Après 3 mois à 25°C | Homogène | | |
| Aspect après 3 mois à 45°C | Homogène | | |
| pH mesuré après 7 jours à 25°C | 6,0 | 6,5 | 6,3 |
| Viscosité mesurée après 7 jours à 25°C | 4.980 mPa.s | 12.300 mPa.s | 28.000 mPa.s |

**Tableau 8**

| Emulsions de type huile dans eau avec émollients | | | |
|---|---|---|---|
| Ingrédients | Proportions massiques | | |
| MONTANOV 68 | 3,0% | 3,0% | 3,0% |
| TRIGLYCERIDES 5545 | 20,0% | 20,0% | 25,0% |
| EMOGREEN^{™} L19 | 5,0% | 10,0% | 0,0% |
| EMOGREEN HP40 | 0,0% | 0,0% | 5,0% |
| Eau déminéralisée | qsp 100,0% | | |
| Polysaccharide (P1) | 1,0% | 0,5% | 1,0% |
| EUXYL PE9010 | 1,0% | 1,0% | 1,0% |

| Aspect visuel des émulsions de type huile-dans-eau | | | |
|---|---|---|---|
| à 25°C | Homogène, Compact Blanc | | |
| Aspect après 3 mois à 25°C | Homogène | | |
| Aspect après 3 mois à 45°C | Homogène | | |
| pH mesuré à après 7 jours à 25°C | 6,1 | 6,5 | 6,5 |
| Viscosité mesurée après 7 jours à 25°C | 53.000 mPa.s | 35.000 mPa.s | 57.000 mPa.s |

**Tableau 9**

| Emulsions de type huile-dans-eau avec huiles végétales | | | | | |
|---|---|---|---|---|---|
| Ingrédients | Proportions massiques | | | | |
| MONTANOV^{™} 68 | 3,0% | 3,0% | 3,0% | 3,0% | 3,0% |
| LANOL^{™} 2681 | 20,0% | 0,0% | 0,0% | 0,0% | 0,0% |
| LANOL^{™} 99 | 0,0% | 20,0% | 0,0% | 0,0% | 0,0% |
| Huile d'amandes douces | 0,0% | 0,0% | 30,0% | 0,0% | 0,0% |
| Huile de jojoba | 0,0% | 0,0% | 0,0% | 30,0% | 0,0% |
| Huile d'olive | 0,0% | 0,0% | 0,0% | 0,0% | 30,0% |
| Eau déminéralisée | qsp 100,0% | | | | |
| Polysaccharide (P1) | 0,0% | 0,5% | 1,0% | 1,0% | 1,0% |
| EUXYL^{™} PE9010 | 1,0% | 1,0% | 1,0% | 1,0% | 1,0% |

| Aspect visuel des émulsions de type huile-dans-eau | | | | | |
|---|---|---|---|---|---|
| à 25°C | Homogène, blanc et lisse | | Hétérogène, blanc, grumeleux | | |
| Après 3 mois à 25°C | Homogène | | | | |
| Après 3 mois à 45°C | Homogène | | | | |
| pH mesuré après 7 jours à 25°C | 6,2 | 6,1 | 6,5 | 6,4 | 6,3 |
| Viscosité mesurée après 7 jours à 25°C (en mPa.s) | 24.500 | 35.000 | 61.000 | 64.000 | 59.000 |

**Tableau 10**

| Emulsions de type huile dans eau | | | | |
|---|---|---|---|---|
| Ingrédients | Quantité massique (%) | | | |
| FLUIDIFEEL^{™} EASY | 3,0% | 3,0% | 3,0% | 3,0% |
| TRIGLYCERIDES 5545 | 20,0% | 20,0% | 10,0% | 10,0% |
| EMOGREEN^{™} L15 | 0,0% | 0,0% | 5,0% | 5,0% |
| Eau déminéralisée | qsp 100,0% | | | |
| Polysaccharide (P1) | 1,0% | 0,5% | 0,5% | 1,0% |
| EUXYL^{™} PE9010 | 1,0% | 1,0% | 1,0% | 1,0% |

| Aspect visuel des émulsions de type huile-dans-eau | | | | |
|---|---|---|---|---|
| Aspect à 25°C | Homogène blanc, et lisse | | | Hétérogène, blanc et grumeleux |
| Aspect après 3 mois à 25°C | Homogène | | | |
| Aspect après 3 mois à 45°C | Homogène | | | |
| pH mesuré après 7 jours à 25°C | 6,4 | 6,4 | 6,6 | 6,4 |
| Viscosité mesurée après 7 jours à 25°C (en mPa.s) | 36.100 | 6.140 | 5.000 | 24.500 |

**Tableau 11**

| Emulsions de type huile dans eau | | | |
|---|---|---|---|
| Ingrédients | Proportions massiques | | |
| FLUIDIFEEL^{™} EASY | 1,0% | 1,0% | 1,0% |
| TRIGLYCERIDES 5545 | 20,0% | 10,0% | 0,0% |
| EMOGREEN^{™} L15 | 0,0% | 5,0% | 0,0% |
| LANOL^{™} 2681 | 0,0% | 0,0% | 15,0% |
| Eau déminéralisée | qsp 100,0% | | |
| Polysaccharide (P1) | 0,5% | 0,5% | 1,0% |
| EUXYL^{™} PE9010 | 1,0% | 1,0% | 1,0% |

| Aspect visuel des émulsions de type huile-dans-eau | | | |
|---|---|---|---|
| A 25°C | Homogène, blanc et lisse | | Hétérogène, blanc et grumeleux |
| Après 3 mois à 25°C | Homogène | | |
| Après 3 mois à 45°C | Homogène | | |
| pH mesuré après 7 jours à 25°C | 6,2 | 6,6 | 6,5 |
| Viscosité mesurée après 7 jours à 25°C | 6.800 mPa.s | 6.500 mPa.s | 23.000 mPa.s |

| Tableau 12]Emulsion hydratante et anti-âge de type huile dans eau | |
|---|---|
| Ingrédients | Proportions massiques |
| Eau déminéralisée | qsp 100,0% |
| Polysaccharide (P1) | 1,0% |
| FLUIDIFEEL^{™} EASY | 3,0% |
| Huile d'amandes douces | 10,0% |
| LANOL^{™} 2681 | 7,0% |
| DUB ZENOAT^{™} | 3,0% |
| AQUAXYL^{™} | 3,0% |
| APAR'AGE^{™} | 2,0% |
| EUXYL^{™} K903 | 1,0% |
| Parfum | 0,1% |
| Acide lactique | qs pH = 5 |

| Aspect visuel des émulsions de type huile-dans-eau | |
|---|---|
| A 25°C | Coulable, couleur ivoire |
| Après 3 mois à 25°C | Homogène |
| Après 3 mois à 45°C | Homogène |
| pH mesuré après 7 jours à 25°C | 5,1 |
| Viscosité mesurée après 7 jours à 25°C | 36300 mPa.s |

**Tableau 13**

| Ingrédients utilisés dans les formulations topiques mentionnées ci-dessus | |
|---|---|
| Nom commercial | Composition ou nom INCI |
| ORAMIX CG110 | Caprylyl-Capryl Glucoside |
| EUXYL K712 | Sodium Benzoate (and) Potassium Sorbate (and) Aqua |
| FLUIDIFEEL^{™} EASY | Lauryl Glucoside (and) Myristyl Glucoside (and) Poly-glyceryl-6 Laurate |
| LANOL^{™} 37T | Triheptanoin |
| EMOGREEN^{™} L19 | C15-19 Alkane |
| NEO HELIOPAN^{™} OS | Salicylate d'éthylhexyle |
| NEO HELIOPAN^{™} BMT | bis-éthylhexyloxyphenol méthoxyphényl triazine |
| NEO HELIOPAN^{™} HMS | Homosalate |
| EUXYL^{™} K903 | Benzyl Alcohol (and) Benzoic Acid (and) Dehydroacetic Acid (and) Tocopherol |
| CONTACTICEL^{™} | Aqua (and) Butylene Glycol (and) Hydrolyzed Rhodophyceae Extract |
| EPHEMER^{™} | Caprylic/Capric Triglyceride - Undaria Pinnatifida Extract |
| TINOSORB^{™} M | Methylene Bis-Benzotriazolyl Tetramethylbutylphenol (and) Aqua (and) Decyl Glucoside (and) Propylene Glycol (and) Xanthan Gum |
| NEO HELIOPAN^{™} HYDRO | Phenylbenzimidazole Sulfonic Acid |
| SENSANOV^{™} WR | C20-22 Alkyl Phosphate (and) C20-22 Alcohols |
| DUB SSIC^{™} | Isocetyl Stearoyl Stearate |
| A15-TiO2-SX-NJE8 | Titanium Dioxide (and) Silica (and) Jojoba Esters |
| EUXYL^{™} PE9010 | Phenoxyethanol (and) Ethylhexylglycerin |
| SENSIVA^{™} PA40 | Phenylpropanol (and) Propanediol (and) Caprylyl Glycol (and) Tocopherol |
| MONTANOV^{™} 68 | Cetearyl Alcohol (and) Cetearyl Glucoside |
| TRIGLYCERIDES 5545 | Caprylic-Capric Triglyceride |

**Tableau 14**

| Ingrédients utilisés dans les formulations topiques mentionnées ci-dessus | |
|---|---|
| Nom commercial | Composition ou nom INCI |
| EMOGREEN^{™} HP40 | C15-19 Alkane (and) Hydrogenated Polyfarnesene |
| LANOL^{™} 2681 | Coco-Caprylate-Caprate |
| LANOL^{™} 99 | Isononyl Isononanoate |
| Huile d'amandes douces | Prunus amygdalus dulcis oil |
| Huile de jojoba | Simmondsia chinensis seed oil |
| Huile d'olive | Olea europaea fruit oil |
| EMOGREEN^{™} L15 | C15-19 Alkane |
| DUB ZENOAT^{™} | Propanediol Dicaprylate |
| AQUAXYL^{™} | Xylitylglucoside (and) Anhydroxylitol (and) Xylitol |
| APAR'AGE^{™} | Water (and) Propanediol (and) Asparagopsis Armata Extract |

## Revendications

1. Procédé de préparation d'un polysaccharide réticulé comprenant les étapes suivantes ;
- Une étape a) de préparation d'un mélange eau - solvant polaire, ledit solvant polaire étant choisi dans le groupe constitué par les alcools aliphatiques comportant de un à quatre atomes de carbone, des cétones comportant de trois à cinq atomes de carbone et les polyols comportant deux ou trois groupes hydroxyle et de deux à six atomes de carbone, ladite préparation étant effectuée en mélangeant l'eau et ledit solvant polaire dans des proportions massiques telles que le rapport massique solvant polaire sur eau est supérieur ou égal à 0,4 et inférieur ou égal à 19,0;
- Une étape b) de dispersion d'au moins un polysaccharide dans ledit mélange eau - solvant polaire préparé à l'étape a), pour obtenir un milieu réactionnel comprenant pour 100% de sa masse, une proportion massique en polysaccharide supérieure à 10% massique et inférieure ou égale à 55% massique, ledit au moins un polysaccharide mis en œuvre à l'étape b), est choisi dans le groupe constitué par la gomme xanthane, le xanthane greffé avec des chaines hydrocarbonées comportant de deux à vingt-deux atomes de carbone et plus particulièrement, la gomme xanthane estérifiée par de l'acide dodécanoïque, les gommes de guar et de konjac, les carraghénanes et plus particulièrement, le kappa-carraghénane et l'iota-carraghénane et des mélanges de deux ou de plusieurs desdits polysaccharides de ce groupe ;
- Une étape c) d'ajustement du pH du milieu réactionnel préparé à l'étape b) à une valeur supérieure ou égale à 8,0 et inférieure ou égale à 13,0 en y ajoutant une base ;
- Une étape d) de réticulation dudit au moins un polysaccharide par addition dans le milieu réactionnel basique obtenu à l'issue de l'étape c), d'un agent de réticulation phosphate choisi dans le groupe constitué du trimétaphosphate de sodium (STMP) et du tripolyphosphate de sodium (STPP), pour obtenir une dispersion basique comprenant du polysaccharide réticulé ;
- Une étape e) d'ajustement du pH de ladite dispersion basique obtenue l'issue de l'étape d), à une valeur inférieure ou égale à 7,0 pour obtenir une dispersion non-basique dudit au moins un polysaccharide réticulé et
- Une étape f) de filtration de ladite dispersion non-basique obtenue à l'issue de l'étape e) pour en récupérer ledit au moins un polysaccharide réticulé attendu, optionnellement suivie :
- Soit d'une étape g) de séchage pour en éliminer les traces de solvants résiduels,
- Soit d'une étape h) d'atomisation dudit au moins un polysaccharide réticulé obtenu à l'étape f) pour en obtenir une poudre.

2. Procédé tel que défini à la revendication 1, dans lequel les étapes a) et b) sont simultanées et constituent une seule étape A) de préparation d'un milieu réactionnel par mélange d'eau, d'un solvant polaire choisi dans le groupe constitué par les alcools aliphatiques comportant de un à quatre atomes de carbone, des cétones comportant de trois à cinq atomes de carbone et les polyols comportant deux ou trois groupes hydroxyle et de deux à six atomes de carbone et d'un polysaccharide en des proportions telles que :
- la proportion massique en ledit au moins un polysaccharide est supérieure à 10% massique et inférieure ou égale à 55% massique dudit milieu réactionnel et que
- le rapport massique solvant polaire sur eau dudit mélange est supérieur ou égal à 0,4 et inférieur ou égal à 19,0.

3. Procédé selon l'une quelconque des revendications 1 à 3, dans lequel ledit solvant polaire du mélange préparé à l'étape a) ou dudit milieu réactionnel préparé à l'étape A), est choisi dans le groupe constitué par le méthanol, l'éthanol, le butanol, l'isopropanol, l'acétone, la méthyl éthyl cétone, le glycérol, le 1,3-propanediol, le butylèneglycol, le 1,3-butanediol, le pentylène glycol l'hexylène glycol et le 2-méthyl 2,4-pentanediol et, plus particulièrement dans le groupe constitué par l'éthanol et l'isopropanol.

4. Procédé tel que défini à l'une quelconque des revendications 1 à 4, dans lequel le rapport massique solvant polaire sur eau dudit mélange préparé à l'étape a) ou dudit milieu réactionnel préparé à l'étape A) est supérieur ou égal à 1,0, et inférieur ou égal à 4,0.

5. Procédé selon l'une quelconque des revendications 1 à 5, dans lequel ledit milieu réactionnel préparé à l'étape b) ou à l'étape A) comprend pour 100% de sa masse, une proportion massique en ledit au moins un polysaccharide supérieure ou égale à 15% et inférieure ou égale à 45% massique.

6. Procédé tel que défini à l'une quelconque des revendications 1 à 6, dans lequel à l'étape c), le pH est ajusté à une valeur supérieure ou égale à 10,0 et inférieure ou égal à 12,5.

7. Procédé tel que défini à l'une quelconque des revendications 1 à 7 dans lequel le rapport massique réticulant STMP ou STPP mis en œuvre à l'étape d) sur ledit au moins un polysaccharide de départ, est supérieur ou égal à 0,0001 et inférieur ou égal à 0,0700 et plus particulièrement supérieur ou égal à 0,0003 et inférieur ou égal à 0,0300.

8. Procédé tel que défini à l'une quelconque des revendications 1 à 8 dans lequel l'agent de réticulation mis en œuvre à l'étape d), est le trimétaphosphate de sodium (STMP).

9. Utilisation d'un polysaccharide réticulé ou d'un mélange de polysaccharides réticulés tel qu'obtenu par le procédé tel que défini à l'une quelconque des revendications 1 à 8, en vue d'épaissir, de stabiliser ou d'émulsionner une formulation topique cosmétique ou pharmaceutique ou d'y suspendre des de particules solides.

10. Formulation topique cosmétique ou pharmaceutique **caractérisée en ce qu'**elle comprend pour 100% de sa masse, de 0,1% à10,0% et plus particulièrement de 0,5% à 5,0% d'un polysaccharide réticulé ou d'un mélange de polysaccharides réticulés tel qu'obtenus par le procédé tel que défini à l'une quelconque des revendications 1 à 8, comme agent épaississant, comme agent stabilisant ou comme agent émulsionnant de ladite formulation topique cosmétique ou pharmaceutique ou comme agent apte et destiné à suspendre des particules solides au sein de ladite formulation topique cosmétique ou pharmaceutique.
